(19) Europäisches Patentamt
European Patent Office
Office européen des brevets

(11) Veröffentlichungsnummer: **0 135 155**
**B1**

(12) # EUROPÄISCHE PATENTSCHRIFT

(45) Veröffentlichungstag der Patentschrift: **26.09.90**

(51) Int. Cl.$^5$: **C 07 D 239/62,**
C 07 D 401/12,
A 61 K 31/515, A 01 N 43/54

(21) Anmeldenummer: **84109765.2**

(22) Anmeldetag: **16.08.84**

(54) **Barbitursäurederivate.**

(30) Priorität: **19.08.83 CH 4535/83**
**20.07.84 CH 3526/84**

(43) Veröffentlichungstag der Anmeldung:
**27.03.85 Patentblatt 85/13**

(45) Bekanntmachung des Hinweises auf die Patenterteilung:
**26.09.90 Patentblatt 90/39**

(84) Benannte Vertragsstaaten:
**AT BE CH DE FR IT LI NL SE**

(56) Entgegenhaltungen:
**EP-A-0 007 541**
**EP-A-0 102 327**
**EP-A-0 105 029**
**EP-A-0 105 030**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Burckhardt, Urs, Dr.**
**Rittergasse 29**
**CH-4051 Basel (CH)**
Erfinder: **Gallay, Jean Jacques, Dr.**
**Blumenrain 19**
**CH-4312 Magden (CH)**
Erfinder: **Kühne, Manfred, Dr.**
**Alemannenweg 11**
**CH-4148 Pfeffingen (CH)**

(74) Vertreter: **Zumstein, Fritz, Dr. et al**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue 5-Phenylcarbamoylbarbitursäurederivate mit anthelmintischer Wirksamkeit und Mittel, die diese Wirkstoffe als Aktivsubstanzen enthalten. Die Wirkstoffe bzw. Mittel eignen sich für die Verwendung bei der Bekämpfung von Helminthen, insbesondere von Nematoden, Cestoden und Trematoden in Haus- und Nutztieren, vor allem in Säugetieren.

Die Erfindung betrifft ferner die Herstellung der neuen Wirkstoffe sowie der sie enthaltenden Mittel.

Die neuen Verbindungen entsprechen der allgemeinen Formel I

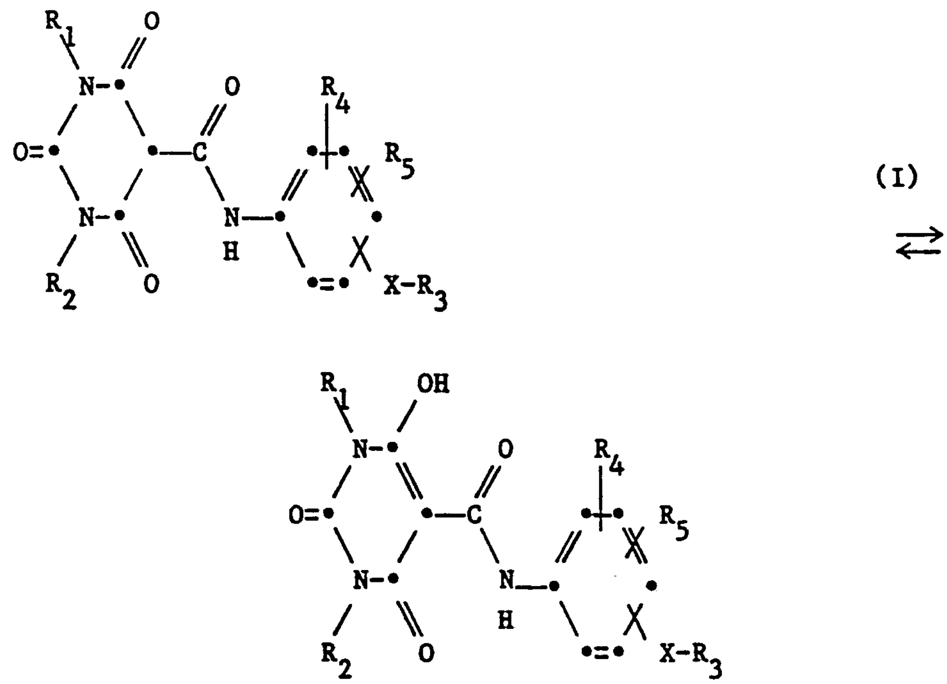

(I)

sowie deren tautomeren Formen und Salzen, worin

$R_1$ und $R_2$ unabhängig voneinander $C_1$—$C_5$-Alkyl, $C_1$—$C_3$-Alkoxy, $C_3$—$C_6$-Cycloalkyl, Allyl oder Phenyl bedeuten;

$R_3$ Phenyl, Naphthyl oder Pyridyl bedeutet; oder $R_3$ ferner bedeutet ein- bis dreifach durch Halogen, Cyano, Thiocyano, Isothiocyano, $C_1$—$C_5$-Alkanoyl, $C_1$—$C_5$-Alkyl, $C_1$—$C_5$-Halogenalkyl, $C_1$—$C_5$-Halogenalkoxy, $C_1$—$C_5$-Alkoxy, Benzyl und/oder durch den Rest Y substituiertes Phenyl oder substituiertes Naphthyl oder substituiertes Pyridyl;

Y ein gegebenenfalls mit $C_1$—$C_3$-Alkyl, $C_1$—$C_3$-Alkoxy, $C_1$—$C_3$-Halogenalkyl oder Halogen substituiertes Benzoyl darstellt;

$R_4$ und $R_5$ unabhängig voneinander Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio bedeuten; und

X für Sauerstoff oder Schwefel steht,

mit der Massgabe, dass

a) $R_1$, $R_2$, $R_4$, $R_5$ und X die für Formel I angegebenen Bedeutungen haben und $R_3$ Naphthyl oder Pyridyl oder ein- bis dreifach durch Halogen, Cyano, Thiocyano, Isothiocyano, $C_1$—$C_5$-Alkanoyl, $C_1$—$C_5$-Alkyl, $C_1$—$C_5$-Halogenalkyl, $C_1$—$C_5$-Halogenalkoxy, $C_1$—$C_5$-Alkoxy, Benzyl und/oder durch den Rest Y substituiertes Naphthyl oder substituiertes Pyridyl bedeutet, wobei Y ein gegebenenfalls mit $C_1$—$C_3$-Alkyl, $C_1$—$C_3$-Alkoxy, $C_1$—$C_3$-Halogenalkyl oder Halogen substituiertes Benzoyl darstellt; oder

b) $R_1$, $R_2$, $R_4$, $R_5$ und X die für Formel I angegebenen Bedeutungen haben und $R_3$ durch Cyano, Thiocyano, Isothiocyano, $C_1$—$C_5$-Alkanoyl, $C_1$—$C_5$-Halogenalkoxy, $C_1$—$C_5$-Alkoxy, Benzyl oder den Rest Y monosubstituiertes oder zusätzlich durch ein oder zwei Substituenten der Gruppe Halogen, Cyano, Thiocyano, Isothiocyano, $C_1$—$C_5$-Alkanoyl, $C_1$—$C_5$-Alkyl, $C_1$—$C_5$-Halogenalkyl, $C_1$—$C_5$-Halogenalkoxy, $C_1$—$C_5$-Alkoxy, Benzyl und den Rest Y substituiertes Phenyl, wobei Y ein gegebenenfalls mit $C_1$—$C_3$-Alkyl, $C_1$—$C_3$-Alkoxy, $C_1$—$C_3$-Halogenalkyl oder Halogen substituiertes Benzoyl darstellt; oder

c) $R_3$, $R_4$, $R_5$ und X die für Formel I angegebenen Bedeutungen haben und einer der Substituenten $R_1$ und $R_2$ für $C_1$—$C_3$-Alkoxy und der andere für $C_1$—$C_5$-Alkyl, $C_1$—$C_3$-Alkoxy, $C_3$—$C_6$-Cycloalkyl, Allyl oder Phenyl steht; oder

d) $R_1$, $R_2$, $R_3$ und X die für Formel I angegebenen Bedeutungen haben und einer der Substituenten $R_4$ und $R_5$ für $C_2$—$C_5$-Alkoxy oder $C_1$—$C_5$-Alkylthio und der andere für Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio steht; oder

e) $R_3$, $R_4$, $R_5$ und X die für Formel I angegebenen Bedeutungen haben und einer der Substituenten $R_1$ und $R_2$ für Alkyl mit 5 Kohlenstoffatomen und der andere für $C_4$—$C_6$-Cycloalkyl oder Phenyl steht; oder

f) $R_1$, $R_2$, $R_3$ und X die für Formel I angegebenen Bedeutungen haben und einer der Substituenten $R_4$ und $R_5$ für in m- oder p-Stellung zur Amidgruppe befindliches Alkyl mit 5 Kohlenstoffatomen steht und der andere Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio bedeutet; oder

g) $R_1$, $R_2$, $R_3$ und X die für Formel I angegebenen Bedeutungen haben und einer der Substituenten $R_4$ und $R_5$ für in o-Stellung zur Amidgruppe befindliches Alkyl mit 5 Kohlenstoffatomen und der andere Halogen, $C_1$—$C_5$-Halogenalkyl, $C_1$—$C_5$-Alkoxy oder $C_1$—$C_5$-Alkylthio bedeutet; oder

h) $R_1$, $R_2$, $R_3$ und X die für Formel I angegebenen Bedeutungen haben, einer der Substituenten $R_4$ und $R_5$ für in o- Stellung zur Amidgruppe befindliches Alkyl mit 5 Kohlenstoffatomen steht und der andere Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio bedeutet, und das Strukturelement —X—$R_3$, wenn es 4-Trifluormethylphenoxy repräsentiert, in o- oder m-Stellung zur Amidgruppe steht; oder

i) einer der Substituenten $R_1$ und $R_2$ für $C_4$—$C_6$-Cycloalkyl oder Phenyl und der andere für $C_1$—$C_5$-Alkyl, $C_1$—$C_5$-Alkoxy, $C_3$—$C_6$-Cycloalkyl, Allyl oder Phenyl steht, einer der Substituenten $R_4$ und $R_5$ für in o-Stellung zur Amidgruppe befindliches Alkyl mit 5 Kohlenstoffatomen und der andere für Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio steht und das Strukturelement —X—$R_3$ 4-Trifluormethylphenoxy repräsentiert.

Besonders erwähnenswerte Verbindungen sind

1,3-Dimethyl-5-[4-(3,5-dichlor-pyridyl-2-oxy)-phenylcarbamoyl]-barbitursäure (Verbindungen Nr. 1.47),

1,3-Dimethyl-5-[4-(5-trifluormethyl-pyridyl-2-oxy)-phenylcarbamoyl]-barbitursäure (Nr. 1.48),

1,3-Dimethyl-5-[3,5-dichlor-4-(3-chlor-5-trifluormethylpyridyl-2-oxy)-phenylcarbamoyl]-barbitursäure (Nr. 1.49),

1,3-Dimethyl-5-[3,5-dichlor-4-(5-trifluormethyl-pyridyl-2-oxy)-phenylcarbamoyl]-barbitursäure (Nr. 1.50),

1-Methyl-3-allyl-5-[4-(5-trifluormethyl-pyridyl-2-oxy)-phenylcarbamoyl (Nr. 151) und

1-Methyl-3-cyclopropyl-5-[4-(5-trifluormethyl-pyridyl-2-oxy)-phenylcarbamoyl]-barbitursäure (Nr. 1.52).

Bevorzugte Einzelverbindungen sind:

1,3-Dimethyl-5-[4-(3,5-dichlor-pyridyl-2-oxy)-phenylcarbamoyl]-barbitursäure,

1,3-Dimethyl-5-[4-(5-trifluormethyl-pyridyl-2-oxy)-phenylcarbamoyl]-barbitursäure,

1-Methoxy-3-methyl-5-[2-isopropyl-4-(4-trifluormethylphenyloxy)-phenylcarbamoyl]-barbitursäure,

1-Methoxy-3-methyl-5-[2-methyl-4-(4-trifluormethylphenoxy)-phenylcarbamoyl]-barbitursäure.

Zu den in Frage kommenden Salzen der Verbindungen der Formel I zählen beispielsweise die Alkalimetall-, Ammonium- oder Aminsalze, wobei Natrium-, Kalium-, Ammonium- oder Alkylaminsalze, besonders Triäthylaminsalze, bevorzugt sind.

Gemäss Formel I sind unter Alkyl als selbständige Gruppe sowie als Teil einer Gruppe von $R_1$ bis $R_5$ gerad- und verzweigtkettiges Alkyl zu verstehen. Dazu zählen die Methyl-, Die Aethyl- sowie die Isomeren der Propyl-, der Butyl- und der Pentylgruppe. Unter Cycloalkyl sind Cyclopropyl-, Cyclobutyl-, Cyclopentyl- sowie Cyclohexylgruppen zu verstehen. Halogen steht für Fluor, Chlor, Brom oder Jod, insbesondere für Fluor oder Chlor. Die Anzahl der Halogenatome in den vorgenannten Halogenalkylresten beträgt 1 bis 5, insbesondere 3.

5-Phenylcarbamoylbarbitursäurederivate sind in der Europäischen Patentanmeldung Nr. 7,541 als Insektizide sowie als Inhibitoren der Entwicklung von Insekten beschrieben. Sie besitzen folgende allgemeine Formel:

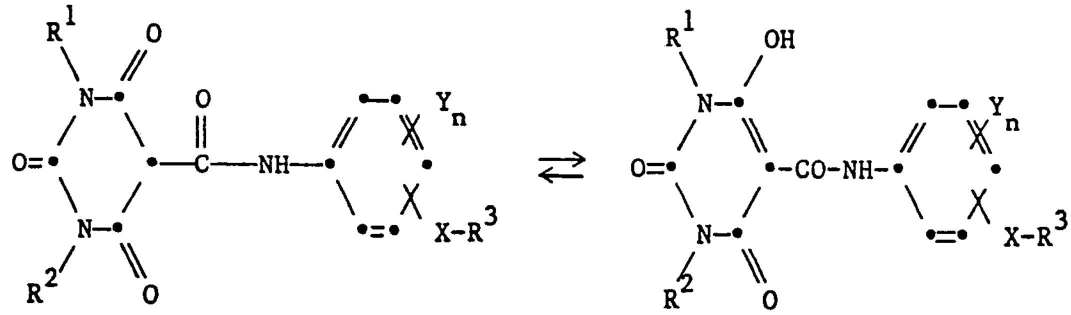

worin

$R^1$ = H, Alkyl

$R^2$ = H, Alkyl

$R^3$ = Halogenalkyl, durch Halogenalkyl substituiertes Phenyl

X = O, S
Y = Halogen, Halogenalkyl
n = 0, 1, 2
X, $R^3$, Y = in ortho-Stellung zueinander gemeinsam auch die Gruppe $—O—CF_2—O—CF_2—$ bedeuten.

Die erfindungsgemässen neuen Wirkstoffe der Formel I unterscheiden sich strukturell in charakteristischer Weise von den aus der EP—OS Nr. 7,541 bekannten Barbitursäurederivaten. Dazu wurde überraschenderweise gefunden, dass die neuen Verbindungen ein günstigeres Wirkungsspektrum gegen im Tierorganismus, vor allem in Säugetieren, parasitierende Helminthen besitzen. So sind sie gegen Nematoden, Cestoden und Trematoden mit gutem Erfolg anwendbar. Dabei zeichnen sie sich vor allem dadurch aus, dass sie auch gegen benzimidazolresistende, insbesondere gegen thiabendazol-resistente Species voll wirksam sind, wobei unter "Thiabendazol" der Wirkstoff 2-[4-Thiazolyl]-benzimidazol zu verstehen ist. Daneben weisen die Wirkstoffe der Formel I ausgeprägte insektizide Eigenschaften auf, die sie insbesondere zur Bekämpfung von keratinvertilgenden Insekten geeignet machen. Weitere 5-Phenylcarbamoylbarbitursäurederivate mit abweichender Struktur werden in den europäischen Patentanmeldungen 105 030, 102 327 und 105 029 offenbart.

Die Wirkstoffe der Formel I werden hergestellt, indem man zur Reaktion bringt:

a) einen Ester der Formel II

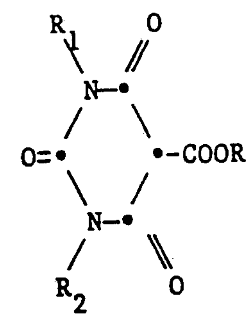

(II)

mit einem Anilinderivat der Formel III

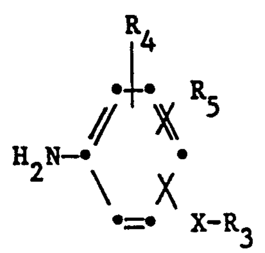

(III)

worin R eine Niederalkyl- oder eine gegebenenfalls durch Nitro substituierte Phenylgruppe darstellt und die Reste $R_1$ bis $R_5$ und X die unter der Formel I angegebenen Bedeutungen besitzen, oder

b) eine substituierte Barbitursäure der Formel IV

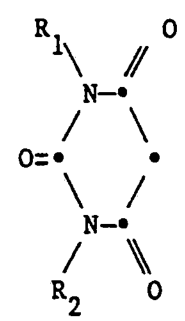

(IV)

mit einem substituierten Phenylisocyanat der Formel (V)

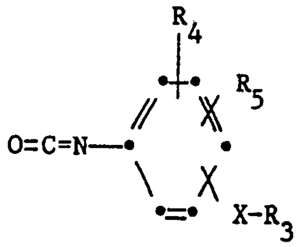

(V)

worin die Reste $R_1$ bis $R_5$ und X die unter Formel I angegebenen Bedeutungen besitzen, oder

c) eine substituierte Barbitursäure der Formel IV mit einem substituierten Benzoylazid der Formel VI

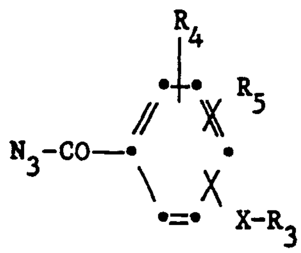

(VI)

worin die Reste $R_1$ bis $R_5$ und X die unter Formel I angegebene Bedeutungen besitzen.

Die Herstellungsvarianten (a) und (c) werden bei Reaktionstemperaturen zwischen 50° und 250°C, vorzugweise 70° bis 220°C, durchgeführt. Variante (b) erfordert Reaktionstemperaturen zwischen 0° und 220°C, insbesondere 0° bis 200°C. Die Reaktionen (a), (b) und (c) können bei normalem oder erhöhtem Druck und in Abwesenheit oder vorzugsweise in Gegenwart reaktionsinerter Lösungs- oder Verdünnungsmittel durchgeführt werden, wobei in manchen Fällen vorteilhafterweise mit einer Base gearbeitet wird.

Die Herstellung der erfindungsgemässen Salze von Verbindungen der Formel I erfolgt durch übliche Neutralisation der freien Säure mit einer Base, insbesondere einer physiologisch verträglichen Base. Vorzugsweise genannt seien Alkalisalze wie Natrium-, Kalium- oder Lithiumsalze sowie Ammoniumsalze und Trialkylaminsalze wie z.B. das bevorzugte Triethylaminsalz. Die Neutralisation wird in einem reaktionsinerten polaren Lösungsmittel, z.B. einem Alkanol, Ester oder einer etherartigen Verbindung, durchgeführt.

Für die Herstellung der erfindungsgemässen Wirksubstanzen geeignete Lösungs- oder Verdünnungsmittel sind z.B. Ether und etherartige Verbindungen wie Dialkylether (Diethylether, Diisopropylether, tert.-Butylmethylether usw.), Anisol, Dioxan, Tetrahydrofuran; aliphatische und aromatische Kohlenwasserstoffe wie Benzol, Toluol, Petrolether; halogenierte Kohlenwasserstoffe wie Chlorbenzol, Methylenchlorid, Chloroform, Ethylenchlorid, Tetrachlorkohlenstoff, Tetrachlorethylen; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Als Basen kommen organische und anorganische Base in Betracht; z.B. vorzugsweise tertiäre Amine wie Trialkylamine (Trimethylamin, Triethylamin, Tripropylamin usw.), Pyridin und Pyridinbasen (z.B. 4-Dimethylaminopyridin, 4-Pyrrolidylaminopyridin usw.), Picoline und Lutidine sowie Oxide, Hydroxide, Carbonate und Hydrogencarbonate von Alkali- und Erdalkalimetallen (z.B. CaO, BaO, NaOH, KOH, $Ca(OH)_2$, $KHCO_3$, $NaHCO_3$, $Ca(HCO_3)_2$, $K_2CO_3$, $Na_2CO_3$ usw.), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.-butylat oder Natriummethylat. Die Base wird vorteilhafterweise in bezug auf die Reaktanden in 10 bis 100% der äquimolaren Menge zugesetzt.

In manchen Fällen kann es von Vorteil sein, wenn die Reaktion unter Schutzgasatmosphäre durchgeführt wird. Geeignete Schutzgase sind z.B. Stickstoff, Helium, Argon oder Kohlendioxid.

Die freie Säure der Formel I führt durch Umsetzung mit Basen zu den ebenfalls zur Erfindung gehörenden Salzen.

Die in den Herstellungsvarianten (a), (b) und (c) genannten Ausgangsstoffe sind bekannt (siehe z.B. Chem. Ber. *54*, 1038 [1921]) oder können in analoger Weise wie die bekannten Substanzen hergestellt werden.

Das beschriebene Herstellungsverfahren ist einschliesslich aller Varianten (a), (b) und (c) ein Bestandteil vorliegender Erfindung.

Die erfindungsgemässen Wirkstoffe der Formel I können in unterschiedlichen tautomeren Formen vorliegen, nämlich in der Keto- oder Enolform oder in einem Gemisch aus Keto- und Enolform. Diese Erfindung betrifft sowohl die einzelnen Tautomeren als auch deren Gemische, aber auch die Salze jeder dieser Formen und deren Herstellung.

Die erfindungsgemäßen Wirkstoffe können in einem Verfahren zum prophylaktischen Schutz von

Tieren gegen parasitäre Helminthen eingesetzt werden, bei welchem man die Wirkstoffe der Formel I bzw. die Wirkstofformulierungen als Zusatz zum Futter oder zu den Tränken oder auch in fester oder flüssiger Form oral, durch Injektion oder mittels der Pour-on-Methode den Tieren appliziert. Darüber hinaus betrifft die Erfindung auch die Verwendung der Wirkstoffe der Formel I zur Bekämpfung von Insekten, insbesondere von keratinvertilgenden Insekten. Diese Verwendung besteht im einzelnen in einem Verfahren zum Schutz von keratinischen bzw. keratinhaltigen Materialien vor dem Befall durch keratinfressende Insekten und vor Insektenfrass, das durch gekennzeichnet ist, dass man das zu schützende Material mit dem Wirkstoff oder einer Wirkstofformulierung behandelt. Ferner betrifft die Erfindung die Wirkstoffe der Formel I bzw. sie enthaltende geeignete Formulierungen als Schutzmittel für keratinisches bzw. keratinhaltiges Material gegen keratinfressende Insekten sowie das mit Hilfe von Verbindungen der Formel I geschützte bzw. ausgerüstete Material.

Bei der Anwendung der erfindungsgemässen Wirkstoffe bzw. der erfindungsgemässen Schädlingsbekämpfungsmittel zur Schädlingsbekämpfung können die Wirkstoffe der Formel I in allen tautomeren Formen, deren Mischungen oder in Form ihrer Salze eingesetzt werden.

Unter den bei Warmblütern vorkommenden Endoparasiten verursachen namentlich die Helminthen grosse Schäden. So zeigen zum Beispiel von diesen Parasiten befallene Tiere nicht nur ein verlangsamtes Wachstum, sonderen teilweise sogar Schädigungen, die zur Mortalität führen können. Daher ist es von grosser Bedeutung, therapeutische Mittel zu entwickeln, die sich zur Bekämpfung von Helminthen und deren Entwicklungsstadien sowie zur Vorbeugung gegen den Befall durch diese Parasiten eignen. Besonders gefährliche Wurmkrankheiten sind solche, die durch im Magen-Darmtrakt und anderen Organen parasitierende Nematoden, Cestoden und Trematoden hervorgerufen werden und vor allem bei Wiederkäuern, wie Schafen, Rindern und Ziegen sowie Pferden, Schweinen, Rotwild, Hunden, Katzen und Geflügel auftreten.

Die durch Helminthiasen verursachten Schäden können bei chronischem und vor allem bei epidemischem Auftreten der Wurmerkrankungen in Viehherden beträchtlich sein. Sie äussern sich unter anderem in Produktivitäts-Verminderungen, geschwächter Widerstandskraft und erhöhter Mortalität. Bekämpfung und Vorbeugung von Helminthiasen gelten deshalb als vordringliche Aufgabe, um derartige vor allem volkswirtschaftlich ins Gewicht fallende Schäden zu vermeiden oder zu mindern.

In der vorliegenden Beschreibung werden unter dem Begriff "Helminthen" insbesondere parasitische Würmer verstanden, die zu den Phyla Platyhelminthes (Cestoden, Trematoden) und Nemathelminthes (Nematodes und Verwandte) gehören, also Bandwürmer, Saugwürmer und Rundwürmer des Gastrointestinal-Traktes und anderer Organe (z.B. Leber, Lunge, Niere, Lymphgefässe, Blut etc.). Es sind zwar eine Reihe von Stoffen mit anthelmintischer Wirkung bekannt, die für die Bekämpfung der verschiedenen Helminthen-species vorgeschlagen wurden. Diese vermögen jedoch nicht voll zu befriedigen, sei es, dass bei verträglicher Dosierung eine Ausschöpfung ihres Wirkungsspektrums nicht möglich ist, oder dass sie in therapeutisch wirksamen Dosen unerwünschte Nebenwirkungen oder Eigenschaften zeigen. In diesem Zusammenhang spielt auch die heute vermehrt auftretende Resistenz gegen bestimmte Stoffklassen eine immer bedeutendere Rolle. Das beispielsweise in der Literatur beschriebene "Albendazol" (British Pat. No. 1464326; Am. J. Vet. Res. *38*, 1425—1426 (1977); Am. J. Vet. Res. *37*, 1515—1516 (1976); Am. J. Vet. Res. *38*, 807—808 (1977); Am. J. Vet. Res. *38*, 1247—1248 (1977)) besitzt zwar ein begrenztes anthelmintisches Wirkungsspektrum bei Wiederkäuern. Seine Wirkung gegen Benzimidazol-resistente Nematoden und adulte Leberegel ist jedoch unzureichend, wobei vor allem die pathologisch wichtigen unreifen Wanderformen der letzteren bei den für das Wirtstier verträglichen Dosierungen nicht angegriffen werden.

Ueberraschenderweise wurde nun festgestellt, dass die Wirkstoffe der Formel I sowohl eine intensive anthelmintische Wirksamkeit mit breitem Wirkungsspektrum gegen Nematoden, Cestoden und Trematoden als auch zusätzlich eine günstige Warmblütertoxizität besitzen.

Die erfindungsgemässen neuen Wirkstoffe der Formel I sind beispielsweise zur Bekämpfung parasitärer Nematoden der Ordnungen (nach K. I. Skrajabin)

Rhabditida
Ascaridida
Spirurida
Trichocephalida

oder zur Bekämpfung von Cestoden der Ordnungen (nach Wardle & McLeod)

Cyclophyllidae
Pseudophyllidae

oder zur Bekämpfung von Trematoden der Ordnung

Digenea

bei Haus- und Nutztieren wie Rindern, Schafen, Ziegen, Pferden, Schweinen, Katzen, Hunden und Geflügel geeignet. Sie können den Tieren sowohl als Einzeldosis als auch wiederholt verabreicht werden, wobei die einzelnen Gaben je nach Tierart vorzugsweise zwischen 1 und 500 mg pro kg Körpergewicht betragen. Durch eine protrahierte Verabreichung wird in manchen Fällen eine bessere Wirkung erzielt oder man kann mit geringeren Gesamtdosen auskommen.

Die erfindungsgemäsen Mittel werden hergestellt, indem die Wirkstoffe der Formel I mit flüssigen und/ oder festen Formulierungshilfsstoffen durch schrittweises Vermischen und/oder Vermahlen derart in

Kontakt gebracht werden, dass eine anwendungskonforme optimale Entfaltung der anthelmintischen Aktivität der Formulierung erzielt wird.

Die Formulierungsschritte können durch Kneten, Granulieren (Granulate) und gegebenenfalls Pressen (Pellets) ergänzt werden.

Als Formulierungshilfsstoffe dienen beispielsweise feste Trägerstoffe, Lösungsmittel und gegebenenfalls oberflächenaktive Stoffe (Tenside).

Zur Bereitung der erfindungsgemässen Mittel werden folgende Formulierungshilfsstoffe verwendet: Feste Trägerstoffe wie z.B. Kaolin, Talkum, Bentonit, Kochsalz, Calciumphosphat, Kohlenhydrate, Cellulosepulver, Baumwollsaatmehl, Polyäthylenglykoläther, gegebenenfalls Bindemittel wie z.B. Gelatine, lösliche Cellulosederivate, gewünschtenfalls unter Zusatz von oberflächenaktiven Stoffen wie ionischen oder nicht-ionischen Dispersionsmitteln; ferner natürliche Gesteinsmehle wie Calcit, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinertes Pflanzenmaterial verwendet werden.

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat; aliphatische Kohlenwasserstoffe wie z.B. Cyclohexan oder Paraffine, Alkohole und Glykole sowie deren Ether und Ester, wie z.B. Ethanol, Ethylenglykol, Ethylenglykolmonomethyl- oder Ethylether, Ketone wie z.B. Cyclohexanon, stark polare Lösungsmittel wie z.B. N-Methyl-2-pyrrolidon, Dimethylsulfoxid oder Dimethylformamid, sowie gegebenenfalls epoxydierte Pflanzenöle wie z.B. epoxydiertes Kokosnussöl oder Sojaöl und Wasser.

Als oberflächenaktive Verbindungen kommen je nach Art des zu formulierenden Wirkstoffes der Formel I nicht-ionogene, kation- und/oder anionaktive Tenside mit guten Emulgier-, Dispergier- und Netzeigenschaften in Betracht. Unter Tensiden sind und Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$—$C_{22}$), wie z.B. die Na- oder K-Salze der Oel- oder Stearinsäure, oder von natürlichen Fettsäuregemischen, die z.B. aus Kokosnuss- oder Talgöl gewonnen werden können.

Häufig werden sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8—22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4—14)-Ethylenoxyd-Adduktes in Frage.

Als nicht-ionische Tenside kommen in erster Linie Polyglykoletherderivate von aliphatischen oder cycloaliphatischen Alkoholen, gesättigten oder ungesättigten Fettsäuren und Alkyphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nicht-ionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht-ionischer Tenside seien Nonylphenolpolyethoxyethanole, Ricinusölpolyglykolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"McCutcheon's Detergents and Emulsifiers Annual" MC Publishing Corp., Ridgewood New Jersey, 1980.

Sisley and Wood, "Encyclopedia of Surface Active Agents", Chemical Publishing Co., Inc. New York, 1980.

Als Bindemittel für Tabletten und Boli kommen chemisch abgewandelte, in Wasser oder Alkohol lösliche, polymere Naturstoffe in Frage, wie Stärke-, Cellulose- oder Protein-derivate (z.B. Methylcellulose, Carboxymethylcellulose, Ethylhydroxyethylcellulose, Proteine wie Zein, Gelatine und dergleichen) sowie synthetische Polymere wie z.B. Polyvinylalkohol, Polyvinylpyrrolidon etc. Ferner sind in Tabletten Füllstoffe, (z.B. Stärke, mikrokristalline Cellulose, Zucker, Milchzucker etc.), Gleitmittel und Sprengmittel enthalten.

Liegen die anthelmintischen Mittel in Form von Futterkonzentraten vor, so dienen als Trägerstoffe z.B. Leistungsfutter, Futtergetreide oder Proteinkonzentrate. Solche Futterkonzentrate oder -mittel können ausser den Wirkstoffen noch Zusatzstoffe, Vitamine, Antibiotika, Chemotherapeutika, oder andere Pestizide, vornehmlich Bakteriostatika, Fungistatika, Coccidiostatika, oder auch Hormonpräparate, Stoffe mit anaboler Wirkung oder das Wachstum begünstigende, die Fleischqualität von Schlachttieren beeinflussende oder in anderer Weise für den Organismus nützliche Stoffe enthalten. Werden die Mittel oder die darin enthaltenen Wirkstoffe der Formel I direkt dem Futter oder den Viehtränken zugesetzt, so enthält das Fertigfutter oder die Fertigtränke die Wirkstoffe vorzugsweise in einer Konzentration von etwa 0,0005 bis 0,02 Gewichtsprozent (5—200 ppm).

Die vorliegende Erfindung schließt auch die Verwendung einer Verbindung der Formel I zur Herstellung eines Arzneimittels zur Bekämpfung tierparasitärer Helminthen ein.

Die Applikation der erfindungsgemässen Mittel an die zu behandelnden Tiere kann peroral, parenteral, subcutan oder topikal durchgeführt werden, wobei die Mittel in Form von Lösungen, Emulsionen, Suspensionen (Drenchs), Pulvern, Tabletten, Boli und Kapseln vorliegen.

Die erfindungsgemässen anthelmintischen Mittel enthalten in der Regel 0,1 bis 99 Gew.%, insbesondere 0,1 bis 95 Gew.-% Wirkstoff der Formel I, 99,9 bis 1 Gew.-%, insbesondere 99,8 bis 5 Gew.-%, eines festen oder flüssigen Zusatzstoffes, darunter 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Solche Mittel können noch weitere Zusätze wie Stabilisatoren, Entschäumer, Viskositätsregulatoren, Bindemittel, Haftmittel sowie andere Wirkstoffe zur Erzielung spezieller Effekte enthalten.

Derartige vom Endverbraucher verwendete anthelmintische und/oder insektizide Mittel sind ebenfalls ein Bestandteil der vorliegenden Erfindung.

Die nachfolgenden Beispiele dienen zur näheren Erläuterung der Erfindung.

1. Herstellungsbeispiele

1.7. 1,3-Dimethyl-5-[4-(3,5-dichlor-pyridyl-2-oxy)-phenylcarbamoyl]-barbitursäure

1,60 g (0,007 Mol) 1,3-Dimethyl-5-ethoxycarbonyl-barbitursäure und 1,79 g (0,007 Mol) 4-(3,5-Dichlor-pyridyl-2-oxy)-anilin werden in 25 ml Toluol suspendiert und 16 Stunden auf Rückflusstemperatur erhitzt, wobei Ethanol entweicht. Nach Abkühlen wird der Niederschlag abfiltriert, mit Aethanol nachgewaschen und getrocknet.

Ausbeute: 2,7 g (89% der Theorie), Smp. 234—235°C.

1.8. 1,3-Dimethyl-5-[4-(5-trifluormethyl-pyridyl-2-oxy)-phenylcarbamoyl]-barbitursäure

1,60 g (0,007 Mol) 1,3-Dimethyl-5-ethoxycarbonyl-barbitursäure und 1,75 g (0,007 Mol) 4-(5-Trifluormethyl-pyridyl-2-oxy)-anilin werden in 20 ml Toluol suspendiert und 16 Stunden auf Rückflusstemperatur erhitzt, wobei Ethanol entweicht. Nach Abkühlen wird der Niederschlag abfiltriert, mit Aethanol nachgewaschen und getrocknet.

Ausbeute: 2,7 g (89% der Theorie), Smp. 180—181°C.

1.9. 1,3-Dimethyl-5-[4-(5-trifluormethyl-pyridyl-2-oxy)-phenylcarbamoyl]-barbitursäure

11,41 g (0,005 Mol) 1,3-Dimethyl-5-ethoxycarbonyl-barbitursäure und 12,7 g (0,050 Mol) 4-(5-Trifluormethyl-pyridyl-2-oxy)-anilin werden in 200 ml Ethanol und 15 ml Dimethylformamid suspendiert und unter Schutzgasstrom (Stickstoff) 18 Stunden auf Rückflusstemperatur erhitzt. Nach Abkühlen wird der Niederschlag abfiltriert, mit Aceton nachgewaschen und getrocknet.

Ausbeute: 19,5 g (90% der Theorie), Smp. 180—181°C.

1.10. 1,3-Dimethyl-5-[4-(3,5-dichlor-pyridyl-2-oxy)-phenylcarbamoyl]-barbitursäure

7,8 g (0,050 Mol) 1,3-Dimethyl-barbitursäure und 14,0 g (0,050 Mol) 4-(3,5-Dichlor-pyridyl-2-oxy)-phenylisocyanat werden in 50 ml Xylol suspendiert und tropfenweise mit 1 g (0,010 Mol) Triethylamin versetzt. Die Temperatur steigt auf 45° bis 50°C. Nach weiterer Zugabe von 50 ml Xylol wird das Gemisch 18 Stunden bei dieser Temperatur gerührt. Dann wird 1/3 des Xylols abdestillert. Nach Abkühlung wird der

Niederschlag abfiltriert, mit Xylol nachgewaschen, mehrmals in HCl-1n suspendiert und anschliessend gründlich mit Wasser gewaschen und getrocknet.

1.11. 1,3-Dimethyl-5-[4-(3,5-dichlor-pyridyl-2-oxy)-phenylcarbamoyl]-barbitursäure

Zu einer Lösung von 4,0 g (0,01 Mol) 4-(3,5-Dichlorpyridyl-2-oxy)benzoylazid in 50 ml Toluol werden 1,56 g (0,01 Mol) 1,3-Dimethylbarbitursäure gegeben. Anschliessend wird eine Lösung von 0,02 g (0,002 Mol) Triäthylamin in 5 ml Toluol bei Raumtemperatur zugetropft. Das Gemisch wird dann 1 Stunde bei 50°C gerührt und danach die Temperatur stufenweise um jeweils 20°C erhöht bis die Rückflusstemperatur erreicht ist. Die Rückflusstemperatur wird bis zur Beendigung der Stickstoffentwicklung aufrechterhalten. Nach Abkühlung wird der ausgefallene Niederschlag abfiltriert, mit Aethanol nachgewaschen, mehrmals in 1N HCl suspendiert und anschliessend gründlich mit Wasser gewaschen und getrocknet.

Ausbeute: 4 g (85% der Theorie), Smp. 234—235°C.

Das als Vorstufenpräparat eingesetzte Benzoylazid wird wie folgt hergestellt:

6,7 g (0,024 Mol) 3,5-Dichlorpyridyl-2-oxybenzoesäure werden in 40 ml Aceton bei 0°C mit 3,4 g (0,031 Mol) Chlorameisensäureäthylester in Gegenwart von 2,9 g (0,028 Mol) Triäthylamin versetzt und weitere 2,4 g (0,036 Mol) Natriumazid in 8 ml Wasser dazu gegeben. Nach dreistündigem Rühren bei 0°C wird das Gemisch in 100 ml Wasser gegossen und mit 60 ml Toluol extrahiert. Die Toluolphase wird abgetrennt, über Natriumsulfat bei 0°C getrocknet und nach Filtration für die oben beschriebene Reaktion verwendet.

1.12. 1,3-Dimethyl-5-[4-(3,5-dichlorpyridyl-2-oxy)-phenylcarbamoyl]-barbitursäure

Eine Suspension von 7,8 g (0,050 Mol) 1,3-Dimethyl-barbitursäure und 18,0 g (0,050 Mol) 4-(3,5-Dichlor-pyridyl-2-oxy)-benzoylazid in 50 ml Xylol werden tropfenweise mit 1 g (0,010 Mol) Triethylamin versetzt. Die Temperatur steigt auf 45° bis 50°C. Nach weiterer Zugabe von 50 ml Xylol wird das Gemisch 18 Stunden bei dieser Temperatur gerührt. Dann wird 1/3 des Xylols abdestilliert. Nach Abkühlung wird der Niederschlag abfiltriert, mit Xylol nachgewaschen, mehrmals in HCl-1n suspendiert und anschliessend gründlich mit Wasser gewaschen und getrocknet.

Ausbeute: 19,0 g (85% der Theorie), Smp. 234—235°C.

TABELLE 1
Verbindung der Formel

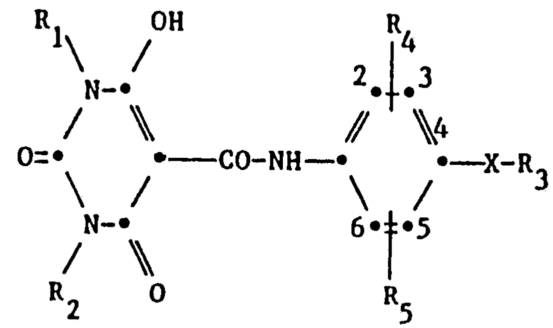

| Verb. Nr. | $R_1$ | $R_2$ | $R_4$ | $R_5$ | X | $R_3$ | Physikal. Konst. [°C] |
|---|---|---|---|---|---|---|---|
| 1.1 | $CH_3$ | $CH_3$ | 2-$CH_3$ | 6-$CH_3$ | O | $C_6H_3CN(2)CF_3(4)$ | Fp. 214—215 |
| 1.2 | $CH_3$ | $CH_3$ | H | H | O | $C_6H_3CN(2)CF_3(4)$ | Fp. 204—207 |
| 1.3 | $CH_3$ | $CH_3$ | 3-$CF_3$ | H | O | $C_6H_4OCF_2H(4)$ | Fp. 148—150 |
| 1.4 | $CH_3$ | $CH_3$ | H | H | O | $C_6H_3Cl(4)OCF_2H(3)$ | Fp. 180—183 |
| 1.5 | $CH_3$ | $CH_3$ | 2-$SCH_3$ | H | O | $C_6H_3Cl(2)CF_3(4)$ | Fp. 195—196 |
| 1.6 | $CH_3$ | $CH_3$ | 2-$CH_3$ | 6-$CH_3$ | O | $C_6H_4$(Benzyl)(4) | Fp. 162—163 |
| 1.7 | $CH_3$ | $CH_3O$ | 2-$CH_3$ | H | O | $C_6H_4CF_3(4)$ | Fp. 164—166 |
| 1.8 | $CH_3$ | $CH_3O$ | 2-$C_3H_7$-i | H | O | $C_6H_4CF_3(4)$ | Fp. 150—152 |
| 1.9 | $CH_3$ | $CH_3$ | H | H | O | (2)-Pyridyl-$Cl_2$(3,5) | Fp. 235—236 |
| 1.10 | $CH_3$ | $CH_3$ | H | H | O | (2)-Pyridyl-$CF_3$(5) | Fp. 180—181 |
| 1.11 | $CH_3$ | $CH_3$ | 3-Cl | 5-Cl | O | (2)-Pyridyl-Cl(3)-$CF_3$(5) | fest |
| 1.12 | $CH_3$ | $CH_3$ | 3-Cl | 5-Cl | O | (2)-Pyridyl-$CF_3$(5) | fest |
| 1.13 | $CH_3$ | $CH_2$—$CH=CH_2$ | H | H | O | (2)-Pyridyl-$CF_3$(5) | Fp. 105—107 |
| 1.14 | $CH_3$ | Cyclopropyl | H | H | O | (2)-Pyridyl-$CF_3$(5) | Fp. 142—144 |

TABELLE 1 (Fortsetzung)

| Verb. Nr. | $R_1$ | $R_2$ | $R_4$ | $R_5$ | X | $R_3$ | Physikal. Konst. [°C] |
|---|---|---|---|---|---|---|---|
| 1.15 | $CH_3O$ | $CH_3$ | H | H | O | $C_6H_3Cl_2(2,4)$ | Fp. 177—178 |
| 1.16 | $CH_3O$ | $CH_3O$ | H | H | O | $C_6H_3Cl_2(2,4)$ | |
| 1.17 | $CH_3O$ | $CH_3$ | H | H | O | $C_6H_4CF_3(3)$ | |
| 1.18 | $CH_3O$ | $CH_3$ | H | H | O | $C_6H_4CF_3(4)$ | Fp. 175—176 |
| 1.19 | $CH_3O$ | $CH_3$ | H | H | O | $C_6H_3Cl(2)CF_3(4)$ | |
| 1.20 | $CH_3O$ | $CH_3O$ | H | H | O | $C_6H_4CF_3(4)$ | |
| 1.21 | $CH_3$ | $CH_3$ | H | H | O | (5)-Pyridyl-Cl(4)$CF_2CCl_2F$(2) | |
| 1.22 | $CH_3$ | $CH_3$ | 3-Cl | H | O | (2)-Pyridyl-Cl(3)$CF_2CCl_2F$(5) | |
| 1.23 | $CH_3$ | $CH_3$ | H | H | O | (2)-Pyridyl-Cl(3)$CF_2CClF_2$(5) | |
| 1.24 | $CH_3$ | $CH_3$ | 2-$CH_3$ | 6-$CH_3$ | O | (2)-Pyridyl-Cl(3)$CF_3$(5) | |
| 1.25 | $CH_3$ | $CH_3$ | H | H | O | (2)-Pyridyl-Cl(3)$CF_3$(5) | |
| 1.26 | $CH_3$ | $CH_3$ | 2-$C_3H_7$-i | H | O | (2)-Pyridyl-$CF_3$(5) | |
| 1.27 | $CH_3$ | $CH_3$ | 2-$CH_3$ | 6-$CH_3$ | O | (2)-Pyridyl-$CF_3$(5) | |
| 1.28 | $CH_3O$ | $CH_3$ | H | H | O | (2)-Pyridyl-$Cl_2$(3,5) | |
| 1.29 | $CH_3O$ | $CH_3$ | H | H | O | (2)-Pyridyl-Cl(3)$CF_3$(5) | |
| 1.30 | $CH_3O$ | $CH_3$ | H | H | O | (2)-Pyridyl-$CF_3$(5) | |
| 1.31 | $CH_3O$ | $CH_3O$ | H | H | O | (2)-Pyridyl-$Cl_2$(3,5) | |
| 1.32 | $CH_3O$ | $CH_3O$ | H | H | O | (2)-Pyridyl-Cl(3)$CF_3$(5) | |
| 1.33 | $CH_3O$ | $CH_3$ | 3-$OCH_3$ | H | O | $C_6H_3Cl(2)CF_3(4)$ | |
| 1.34 | $CH_3O$ | $CH_3O$ | 3-$OCH_3$ | H | O | $C_6H_3Cl(2)CF_3(4)$ | |

TABELLE 1 (Fortsetzung)

| Verb. Nr. | $R_1$ | $R_2$ | $R_4$ | $R_5$ | X | $R_3$ | Physikal. Konst. [°C] |
|---|---|---|---|---|---|---|---|
| 1.35 | $CH_3$ | $CH_3$ | 3-$OCH_3$ | H | O | (2)-Pyridyl-$Cl_2$(3,5) | |
| 1.36 | $CH_3O$ | $CH_3$ | 3-$OCH_3$ | H | O | (2)-Pyridyl-$Cl_2$(3,5) | |
| 1.37 | $CH_3$ | $CH_3$ | 2-$CH_3$ | 6-$CH_3$ | O | (2)-Pyridyl-$Cl_3$(3,5) | Fp. 251—253 |

TABELLE 2
Verbindung der Formel

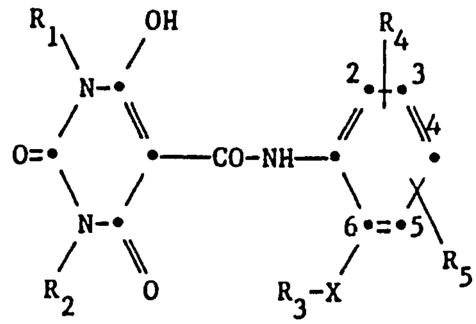

| Verb. Nr. | $R_1$ | $R_2$ | $R_4$ | $R_5$ | X | $R_3$ | Physikal. Konst. [°C] |
|---|---|---|---|---|---|---|---|
| 2.1 | $CH_3$ | $CH_3$ | 2-$CH_3$ | H | O | $C_6H_3CN(2)Cl(4)$ | Fp. 204—206 |
| 2.2 | $CH_3$ | $CH_3$ | H | H | O | $C_6H_3(CN)_2(2,3)$ | Fp. 253—255 |
| 2.3 | $CH_3$ | $CH_3$ | 3-Cl | H | O | $C_6H_3OCH_3(3)Cl(4)$ | Fp. 215—217 |
| 2.4 | $CH_3$ | $CH_3$ | 3-Cl | H | O | $C_6H_3OCH_3(3)Cl(4)$ | Fp. 230—232 |
| 2.5 | $CH_3$ | $CH_3$ | 3-Br | H | O | $C_6H_3OCH_3(3)Cl(4)$ | Fp. 225—227 |
| 2.6 | $CH_3$ | $CH_3$ | 4-Cl | 3-$CF_3$ | O | $C_6H_3OCH_3(3)Cl(4)$ | Fp. 202 |
| 2.7 | $CH_3$ | $CH_3$ | H | H | S | $C_6H_3(CN)_2(2,3)$ | Fp. 246—248 |
| 2.8 | $CH_3$ | $CH_3$ | 3-$CF_3$ | H | O | β-Naphthyl | Fp. 233—234 |
| 2.9 | $CH_3$ | $CH_3$ | 3-Cl | H | O | α-Naphthyl | Fp. 201—202 |
| 2.10 | $CH_3$ | $CH_3$ | 3-Cl | H | O | β-Naphthyl | Fp. 222—223 |
| 2.11 | $CH_3$ | $CH_3$ | 4-Cl | H | O | α-Naphthyl | Fp. 190—191 |
| 2.12 | $CH_3$ | $CH_3$ | 4-Cl | H | O | β-Naphthyl | Fp. 235—237 |
| 2.13 | $CH_3$ | $CH_3$ | 2-Cl | 3-Cl | O | (2)-Pyridyl | Fp. 241—242 |
| 2.14 | $CH_3O$ | $CH_3$ | 4-Cl | H | O | $C_6H_4Cl(4)$ | Fp. 228—230 |
| 2.15 | $CH_3O$ | $CH_3$ | H | H | O | $C_6H_3Cl_2(2,4)$ | Fp. 222—224 |
| 2.16 | $CH_3$ | $CH_3$ | H | H | O | (2)-Pyridyl-$CF_3(5)$ | |

TABELLE 3
Verbindungen der Formel

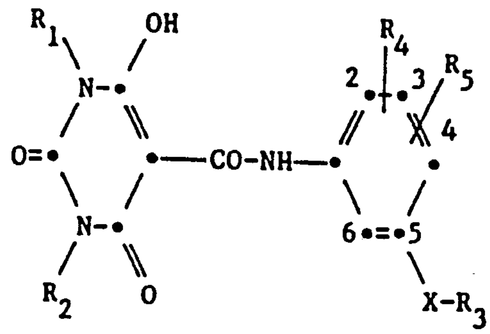

| Verb. Nr. | $R_1$ | $R_2$ | $R_4$ | $R_5$ | X | $R_3$ | Physikal. Konst. [°C] |
|---|---|---|---|---|---|---|---|
| 3.1 | $CH_3$ | $CH_3$ | H | H | O | $C_6H_3CN(2)CF_3(4)$ | Fp. 151 |
| 3.2 | $CH_3$ | $CH_3$ | 2-$CH_3$ | H | O | $C_6H_3CN(2)Cl(4)$ | Fp. 204—206 |
| 3.3 | $CH_3$ | $CH_3$ | H | H | O | (2)-Pyridyl-Cl(3)$CF_3$(5) | |
| 3.4 | $CH_3$ | $CH_3$ | 4-$CH_3$ | H | O | (2)-Pyridyl-Cl(3)$CF_3$(5) | |
| 3.5 | $CH_3O$ | $CH_3$ | 4-$OCH_3$ | H | O | $C_6H_3Cl(2)CF_3(4)$ | |
| 3.6 | $CH_3O$ | $CH_3O$ | 4-$OCH_3$ | H | O | $C_6H_3Cl(2)CF_3(4)$ | |
| 3.7 | $CH_3$ | $CH_3$ | 4-$OCH_3$ | H | O | (2)-Pyridyl-Cl(3)$CF_3$(5) | |
| 3.8 | $CH_3$ | $CH_3$ | 4-$OCH_3$ | H | O | (2)-Pyridyl-$Cl_2$(3,5) | |
| 3.9 | $CH_3O$ | $CH_3$ | 4-$OCH_3$ | H | O | (2)-Pyridyl-$Cl_2$(3,5) | |
| 3.10 | $CH_3$ | $CH_3$ | 4-$OCH_3$ | H | O | (2)-Pyridyl-$CF_3$(5) | |

2. Formulierungsbeispiele (% = Gewichtsprozent)

2.1. Emulsions-Konzentrate

| | a) | b) | c) |
|---|---|---|---|
| Wirkstoff aus Tabelle I | 25% | 40% | 50% |
| Ca-Dodecylbenzolsulfonat | 5% | 8% | 6% |
| Ricinusöl-polyethylenglykolether (36 Mol Ethylenoxid) | 5% | — | — |
| Tributylphenol-polyethylenglykolether (30 Mol Ethylenoxid) | — | 12% | 4% |
| Cyclohexanon | — | 15% | 20% |
| Xylolgemisch | 65% | 25% | 20% |

Aus solchen Konzentraten können durch Verdünnen mit Wasser Emulsionen jeder gewünschten Konzentration hergestellt werden.

2.2. Lösungen

| | a) | b) | c) | d) |
|---|---|---|---|---|
| Wirkstoff aus Tabelle I | 80% | 10% | 5% | 95% |
| Ethylenglykol-monomethyl-ether | 20% | — | — | — |
| Polyethylenglykol MG 400 | — | 70% | — | — |
| N-Methyl-2-pyrrolidon | — | 20 | — | — |
| Epoxydiertes Kokosnussöl | — | — | 1% | 5% |
| Benzin (Siedegrenze 160—190°C) | — | — | 94% | — |

(MG = Molekulargewicht)

Die Lösungen sind zur Anwendung in Form kleinster Tropfen geeignet.

2.3. Granulate

| | a) | b) |
|---|---|---|
| Wirkstoff der Tabelle I | 5% | 10% |
| Kaolin | 94% | — |
| Hochdisperse Kieselsäure | 1% | — |
| Attapulgit | — | 90% |

Der Wirkstoff wird in Methylenchlorid gelöst, auf den Träger aufgesprüht und das Lösungsmittel anschliessend im Vakuum eingedampft. Solche Granulate können dem Viehfutter beigemischt werden.

2.4. Stäubemittel

| | a) | b) |
|---|---|---|
| Wirkstoff der Tabelle I | 2% | 5% |
| Hochdisperse Kieselsäure | 1% | 5% |
| Talkum | 97% | — |
| Kaolin | — | 90% |

Durch inniges Vermischen auf Trägerstoffe mit dem Wirkstoff erhält man gebrauchsfertige Stäubemittel.

2.5. In Wasser dispergierbare Pulvermischung

| | a) | b) | c) |
|---|---|---|---|
| Wirkstoff aus Tabelle I | 25% | 50% | 75% |
| Na-Ligninsulfonat | 5% | 5% | — |
| Oelsäure | 3% | — | 5% |
| Na-Diisobutylnaphthalinsulfonat | — | 6% | 10% |
| Octylphenolpolyethylenglykolether (7—8 Mol Ethylenoxid) | — | 2% | — |
| Hochdisperse Kieselsäure | 5% | 10% | 10% |
| Kaolin | 62% | 27% | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2.6. Emulsions-Konzentrat

| | a) | b) | c) |
|---|---|---|---|
| Wirkstoff aus Tabelle I | 10% | 8% | 60% |
| Octylphenolpolyethylenglykolether (4—5 Mol Ethylenoxid) | 3% | 3% | 2% |
| Ca-Dodecylbenzolsulfonat | 3% | 4% | 4% |
| Ricinusölpolyglykolether (35 Mol Ethylenoxid) | 4% | 5% | 4% |
| Cyclohexanon | 30% | 40% | 15% |
| Xylolgemisch | 50% | 40% | 15% |

Aus diesem Konzentrat können durch Verdünnen mit Wasser Emulsionen jeder gewünschten Konzentration hergestellt werden.

2.7. Stäubemittel

| | a) | b) |
|---|---|---|
| Wirkstoff aus Tabelle I | 5% | 8% |
| Talkum | 95% | — |
| Kaolin | — | 92% |

Man erhält anwendungsfertige Stäubemittel, indem der Wirkstoff mit dem Träger vermischt auf einer geeigneten Mühle vermahlen wird.

2.8. Granulat

| | |
|---|---|
| Wirkstoff aus Tabelle I | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

2.9. Granulat

| | |
|---|---|
| Wirkstoff aus Tabelle I | 3% |
| Polyethylenglykol (MG 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.10. Suspensions-Konzentrat

| | |
|---|---|
| Wirkstoff aus Tabelle I | 40% |
| Ethylenglykol | 10% |
| Nonylphenolpolyethylenglykolether (15 Mol Ethylenoxid) | 6% |

| | |
|---|---|
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspension-Konzentrat, aus welchem durch Verdünnen mit Wasser Suspensionen jeder gewünschten Konzentration hergestellt werden können.

2.11. Tabletten bzw. Boli

| | | |
|---|---|---|
| I | Wirkstoff der Tabelle I | 33,0% |
| | Methylcellulose | 0,80% |
| | Kieselsäure hochdispers | 0,80% |
| | Maisstärke | 8,40% |
| II | Milchzucker krist. | 22,50% |
| | Maisstärke | 17,00% |
| | mikrokrist. Cellulose | 16,50% |
| | Magnesiumstearat | 1,00% |

I Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb 12 M granulieren und trocknen.
II Alle 4 Hilfsstoffe gut mischen.
III Phasen I und II mischen und zu Tabletten oder Boli verpressen.

3. Biologische Beispiele
Die anthelmintische Wirksamkeit wird anhand folgender Versuche demonstriert:

3.1. Versuch an mit Nematoden wie Haemonchus contortus und Trichostrongylus colubriformis infizierten Schafen
Der Wirkstoff wird in Form einer Suspension mit einer Magensonde oder durch Pansen-Injektion Schafen verabreicht, die vorher mit Nematoden wie Haemonchus contortus und Trichostrongylus colubriformis künstlich infiziert wurden. Pro Versuch resp. pro Dosis werden 1 bis 3 Tiere verwendet. Jedes Schaf wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen werden.

Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich.

Shafe, die mit einer Suspension eines Wirkstoffes aus den Tabellen 1 bis 3 behandelt werden, zeigen im Vergleich zu unbehandelten, aber infizierten Vergleichsgruppen bei einer Dosis von weniger als 20 mg/kg Körpergewicht einen um mindestens 90% reduzierten Nematodenbefall. Darüber hinaus zeigen die Verbindungen Nr. 2.11 bei einer Dosis von 12 mg/kg, die Verbindungen Nr. 1.2 und 1.9, bei einer Dosis von 5 mg/kg eine 95 %ige und die Verbindungen Nr. 1.7, 1.8 und 1.10 bei einer Dosis von 2,5 mg/kg eine 100 %ige Wirksamkeit.

3.2. Versuch an mit Fasciola hepatica infizierten Schafen
Der Wirkstoff wird in Form einer Suspension mit einer Magensonde oder durch Pansen-Injektion Schafen verabreicht, die vorher mit Fasciola hepatica künstlich infiziert wurden. Pro Versuch resp. pro Dosis werden 3 Tiere verwendet. Jedes Tier wird mit nur einer einzigen Dosis behandelt.

Eine erste Evaluierung erfolgt dadurch, dass die Anzahl der vor und nach der Behandlung im Kot der Schafe ausgeschiedenen Wurmeier verglichen werden.

Drei bis vier Wochen nach der Behandlung werden die Schafe getötet und seziert. Die Auswertung erfolgt durch das Auszählen der nach der Behandlung in den Gallengängen zurückgebliebenen Leberegel. Gleichzeitig und gleichartig infizierte aber unbehandelte Schafe dienen als Kontrolle resp. Vergleich. Der

Unterschied der in den beiden Gruppen festgestellten Anzahl von Leberegeln ergibt den Wirkungsgrad des geprüften Wirkstoffs.

In diesem Versuch zeigt beispielsweise die Verbindung Nr. 1.8 aus der Tabelle 1 bei einer Dosis von 12 mg/kg Körpergewicht eine mindestens 90 %ige Wirksamkeit gegen Fasciola hepatica.

**Patentansprüche für die Vertragsstaaten: BE CH DE FR IT LI NL SE**

1. 5-Phenylcarbamoylbarbitursäurederivate der allgemeinen Formel I

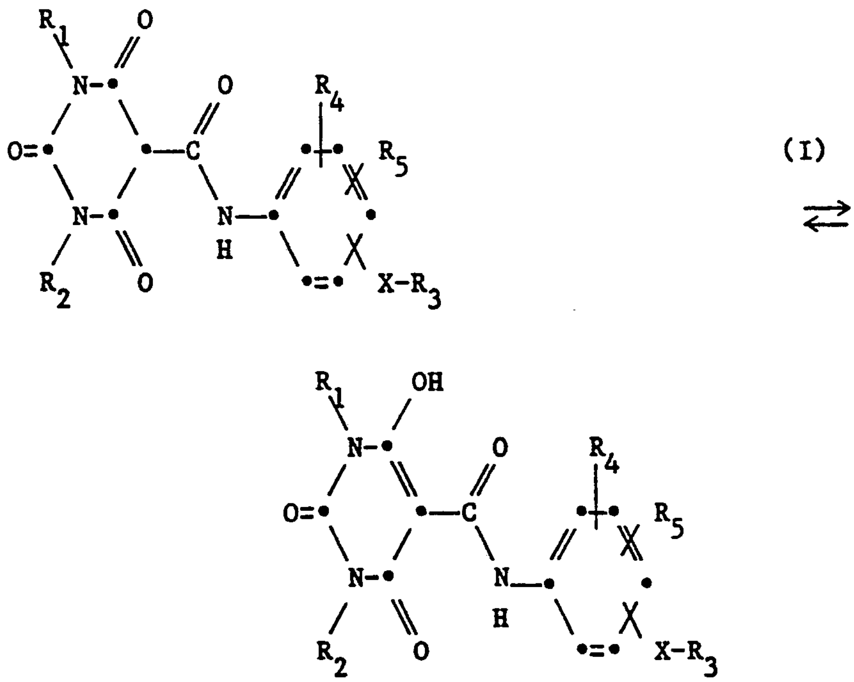

(I)

sowie deren tautomeren Formen und Salze, worin

$R_1$ und $R_2$ unabhängig voneinander $C_1$—$C_5$-Alkyl, $C_1$—$C_3$-Alkoxy, $C_3$—$C_6$-Cycloalkyl, Allyl oder Phenyl bedeuten;

$R_3$ Phenyl, Naphthyl oder Pyridyl bedeutet; oder $R_3$ ferner bedeutet eine- bis dreifach durch Halogen, Cyano, Thiocyano, Isothiocyano, $C_1$—$C_5$-Alkanoyl, $C_1$—$C_5$-Alkyl, $C_1$—$C_5$-Halogenalkyl, $C_1$—$C_5$-Halogenalkoxy, $C_1$—$C_5$-Alkoxy, Benzyl und/oder durch den Rest Y substituiertes Phenyl oder substituiertes Naphthyl oder substituiertes Pyridyl;

Y ein gegebenenfalls mit $C_1$—$C_3$-Alkyl, $C_1$—$C_3$-Alkoxy, $C_1$—$C_3$-Halogenalkyl oder Halogen substituiertes Benzoyl darstellt;

$R_4$ und $R_5$ unabhängig voneinander Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio, bedeuten; und

X für Sauerstoff oder Schwefel steht,

mit der Massgabe, dass

a) $R_1$, $R_2$, $R_4$, $R_5$ und X die für Formel I angegebenen Bedeutungen haben und $R_3$ Naphthyl oder Pyridyl oder ein- bis dreifach durch Halogen, Cyano, Thiocyano, Isothiocyano, $C_1$—$C_5$-Alkanoyl, $C_1$—$C_5$-Alkyl, $C_1$—$C_5$-Halogenalkyl, $C_1$—$C_5$-Halogenalkoxy, $C_1$—$C_5$-Alkoxy, Benzyl und/oder durch den Rest Y substituiertes Naphthyl oder substituiertes Pyridyl bedeutet, wobei Y ein gegebenenfalls mit $C_1$—$C_3$-Alkyl, $C_1$—$C_3$-Alkoxy, $C_1$—$C_3$-Halogenalkyl oder Halogen substituiertes Benzoyl darstellt; oder

b) $R_1$, $R_2$, $R_4$, $R_5$ und X die für Formel I angegebenen Bedeutungen haben und $R_3$ durch Cyano, Thiocyano, Isothiocyano, $C_1$—$C_5$-Alkanoyl, $C_1$—$C_5$-Halogenalkoxy, $C_1$—$C_5$-Alkoxy, Benzyl oder den Rest Y monosubstituiertes oder zusätzlich durch eine oder zwei Substituenten der Gruppe Halogen, Cyano, Thiocyano, Isothiocyano, $C_1$—$C_5$-Alkanoyl, $C_1$—$C_5$-Alkyl, $C_1$—$C_5$-Halogenalkyl, $C_1$—$C_5$-Halogenalkoxy, $C_1$—$C_5$-Alkoxy, Benzyl und den Rest Y substituiertes Phenyl, wobei Y ein gegebenenfalls mit $C_1$—$C_3$-Alkyl, $C_1$—$C_3$-Alkoxy, $C_1$—$C_3$-Halogenalkyl oder Halogen substituiertes Benzoyl darstellt; oder

c) $R_3$, $R_4$, $R_5$ und X die für Formel I angegebenen Bedeutungen haben und einer der Substituenten $R_1$ und $R_2$ für $C_1$—$C_3$-Alkoxy und der andere für $C_1$—$C_5$-Alkyl, $C_1$—$C_3$-Alkoxy, $C_3$—$C_6$-Cycloalkyl, Allyl oder Phenyl steht; oder

d) $R_1$, $R_2$, $R_3$ und X die für Formel I angegebenen Bedeutungen haben und einer der Substituenten $R_4$ und $R_5$ für $C_2$—$C_5$-Alkoxy oder $C_1$—$C_5$-Alkylthio und der andere für Wasserstoff, Halogen, $C_1$—$C_5$-

Halogenalkyl, $C_1$—$C_5$-Alkyl, $C_1$—$C_5$-Alkoxy oder $C_1$—$C_5$-Alkylthio steht; oder

e) $R_3$, $R_4$, $R_5$ und X die für Formel I angegebenen Bedeutungen haben und einer der Substituenten $R_1$ und $R_2$ für Alkyl mit 5 Kohlenstoffatomen und der andere für $C_4$—$C_6$-Cycloalkyl oder Phenyl steht; oder

f) $R_1$, $R_2$, $R_3$ und X die für Formel I angegebenen Bedeutungen haben und einer der Substituenten $R_4$ und $R_5$ für in m- oder p-Stellung zur Amidgruppe befindliches Alkyl mit 5 Kohlenstoffatomen steht und der andere Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio bedeutet; oder

g) $R_1$, $R_2$, $R_3$ und X die für Formel I angegebenen Bedeutungen haben und einer der Substituenten $R_4$ und $R_5$ für in o-Stellung zur Amidgruppe befindliches Alkyl mit 5 Kohlenstoffatomen und der andere Halogen, $C_1$—$C_5$-Halogenalkyl, $C_1$—$C_5$-Alkoxy oder $C_1$—$C_5$-Alkylthio bedeutet; oder

h) $R_1$, $R_2$, $R_3$ und X die für Formel I angegebenen Bedeutungen haben, einer der Substituenten $R_4$ und $R_5$ für in o- Stellung zur Amidgruppe befindliches Alkyl mit 5 Kohlenstoffatomen steht und der andere Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio bedeutet; und das Strukturelement —X—$R_3$, wenn es 4-Trifluormethylphenoxy repräsentiert, in o- oder m-Stellung zur Amidgruppe steht; oder

i) einer der Substituenten $R_1$ und $R_2$ für $C_4$—$C_6$-Cycloalkyl oder Phenyl und der andere für $C_1$—$C_5$-Alkyl, $C_1$—$C_5$-Alkoxy, $C_3$—$C_6$-Cycloalkyl, Allyl oder Phenyl steht, einer der Substituenten $R_4$ und $R_5$ für in o-Stellung zur Amidgruppe befindliches Alkyl mit 5 Kohlenstoffatomen und der andere für Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio steht und das Strukturelement —X—$R_3$ 4-Trifluormethylphenoxy repräsentiert.

2. 1,3-Dimethyl-5-[4-(3,5-dichlor-pyridyl-2-oxy)-phenylcarbamoyl]-barbitursäure.

3. 1,3-Dimethyl-5-[4-(5-trifluormethyl-pyridyl-2-oxy)phenylcarbamoyl]-barbitursäure.

4. 1,3-Dimethyl-5-[3,5-dichlor-4-(3-chlor-5-trifluormethyl-pyridyl-2-oxy)phenylcarbamoyl]-barbitursäure.

5. 1,3-Dimethyl-5-[3,5-dichlor-4-(5-trifluormethyl-pyridyl-2-oxy)-phenylcarbamoyl]-barbitursäure.

6. 1-Methyl-3-allyl-5-[4-(5-trifluormethyl-pyridyl-2-oxy)-phenylcarbamoyl]-barbitursäure.

7. 1-Methyl-3-cyclopropyl-5-[4-(5-trifluormethyl-pyridyl-2-oxy)-phenylcarbamoyl]-barbitursäure.

8. Verfahren zur Herstellung von Verbindungen der Formel I gemäss Anspruch 1, dadurch gekennzeichnet, dass man

a) bei Temperaturen von 50° bis 250°C einen Ester der Formel

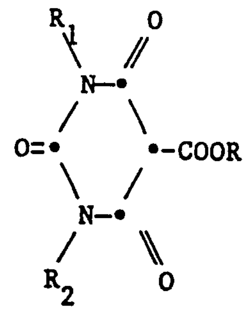

(II)

mit einem Anilinderivat der Formel III

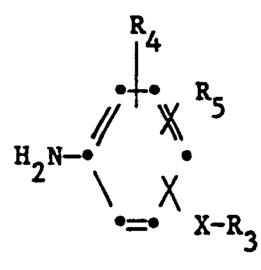

(III)

worin R eine Niederalkyl- oder eine gegebenenfalls durch Nitro substituierte Phenylgruppe darstellt und die Reste $R_1$ bis $R_5$ und X die unter der Formel I in Anspruch 1 angegebenen Bedeutungen besitzen, umsetzt oder

b) bei Temperaturen von 0° bis 220°C eine substituierte Barbitursäure der Formel IV

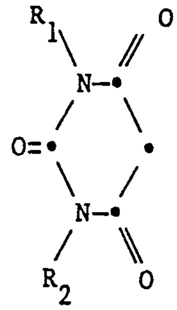

(IV)

mit einem substituierten Phenylisocyanat der Formel V

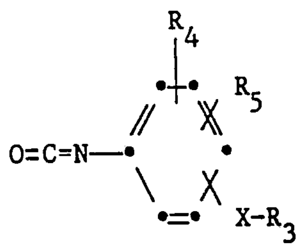

(V)

worin die Reste $R_1$ bis $R_5$ und X die unter Formel I in Anspruch 1 angegebenen Bedeutungen besitzen, umsetzt oder

c) bei Temperaturen von 50° bis 250°C eine substituierte Barbitursäure der Formel IV

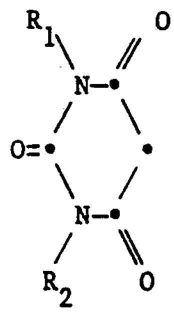

(IV)

mit einem substituierten Benzoylazid der Formel

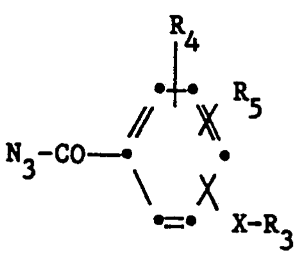

(VI)

worin die Reste $R_1$ bis $R_5$ und X die unter Formel I in Anspruch 1 angegebenen Bedeutungen besitzen, umsetzt.

9. Verfahren nach Anspruch 8, dadurch gekennzeichnet, dass die Verfahren (a), (b) und (c) in Gegenwart reaktionsinerter Lösungs- oder Verdünnungsmittel durchgeführt werden.

10. Verfahren nach den Ansprüchen 8 und 9, dadurch gekennzeichnet, dass die Verfahren (a) und (c) bei 100° bis 220°C und Verfahren (b) bei 0° bis 200°C durchgeführt werden.

11. Verfahren nach den Ansprüchen 9 und 10, dadurch gekennzeichnet, dass die Verfahren (a), (b) und (c) in Gegenwart einer Base durchgeführt werden.

12. Anthelmintisches Mittel, dadurch gekennzeichnet, dass es als aktive Komponente mindestens eine

Verbindung der Formel I, ein Tautomeres oder ein Salz davon gemäss Anspruch 1 neben Träger- und weiteren Hilfsstoffen enthält.

13. Mittel nach Anspruch 12, dadurch gekennzeichnet, dass es einen Wirkstoffgehalt von 0,1 bis 99,0 Gew.% und einen Gehalt an Träger- und weiteren Hilfsstoffen von 99,9 bis 1 Gew.% bestizt.

14. Verbindung der Formel I gemäss Anspruch 1 zur Anwendung bei der Bekämpfung parasitärer Helminthen.

15. Verwendung einer Verbindung der Formel I gemäss Anspruch 1 zur Herstellung eines Arzneimittels zur Bekämpfung tierparasitärer Helminthen.

**Patentansprüche für den Vertragsstaat: AT**

1. Verfahren zur Herstellung von 5-Phenylcarbamoylbarbitursäurederivaten der allgemeinen Formel I

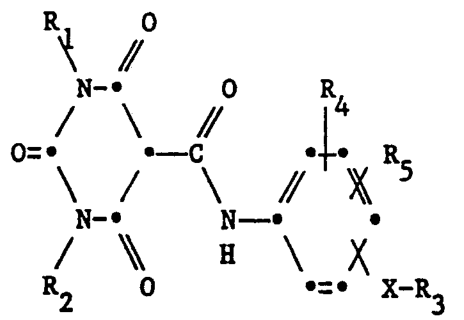

(I)

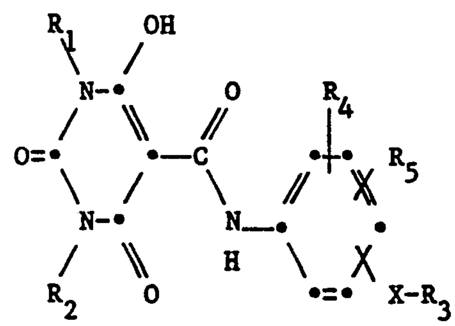

sowie deren tautomeren Formen und Salzen, worin

$R_1$ und $R_2$ unabhängig voneinander $C_1$—$C_5$-Alkyl, $C_1$—$C_3$-Alkoxy, $C_3$—$C_6$-Cycloalkyl, Allyl oder Phenyl bedeuten;

$R_3$ Phenyl, Naphthyl oder Pyridyl bedeutet; oder $R_3$ ferner bedeutet eine- bis dreifach durch Halogen, Cyano, Thiocyano, Isothiocyano, $C_1$—$C_5$-Alkanoyl, $C_1$—$C_5$-Alkyl, $C_1$—$C_5$-Halogenalkyl, $C_1$—$C_5$-Halogenalkoxy, $C_1$—$C_5$-Alkoxy, Benzyl und/oder durch den Rest Y substituiertes Phenyl oder substituiertes Naphthyl oder substituiertes Pyridyl;

Y ein gegebenenfalls mit $C_1$—$C_3$-Alkyl, $C_1$—$C_3$-Alkoxy, $C_1$—$C_3$-Halogenalkyl oder Halogen substituiertes Benzoyl darstellt;

$R_4$ und $R_5$ unabhängig voneinander Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio, bedeuten; und

X für Sauerstoff oder Schwefel steht,

mit der Massgabe, dass

a) $R_1$, $R_2$, $R_4$, $R_5$ und X die für Formel I angegebenen Bedeutungen haben und $R_3$ Naphthyl oder Pyridyl oder ein- bis dreifach durch Halogen, Cyano, Thiocyano, Isothiocyano, $C_1$—$C_5$-Alkanoyl, $C_1$—$C_5$-Alkyl, $C_1$—$C_5$-Halogenalkyl, $C_1$—$C_5$-Halogenalkoxy, $C_1$—$C_5$-Alkoxy, Benzyl und/oder durch den Rest Y substituiertes Naphthyl oder substituiertes Pyridyl bedeutet, wobei Y ein gegebenenfalls mit $C_1$—$C_3$-Alkyl, $C_1$—$C_3$-Alkoxy, $C_1$—$C_3$-Halogenalkyl oder Halogen substituiertes Benzoyl darstellt; oder

b) $R_1$, $R_2$, $R_4$, $R_5$ und X die für Formel I angegebenen Bedeutungen haben und $R_3$ durch Cyano, Thiocyano, Isothiocyano, $C_1$—$C_5$-Alkanoyl, $C_1$—$C_5$-Halogenalkoxy, $C_1$—$C_5$-Alkoxy, Benzyl oder den Rest Y monosubstituiertes oder zusätzlich durch eine oder zwei Substituenten der Gruppe Halogen, Cyano, Thiocyano, Isothiocyano, $C_1$—$C_5$-Alkanoyl, $C_1$—$C_5$-Alkyl, $C_1$—$C_5$-Halogenalkyl, $C_1$—$C_5$-Halogenalkoxy, $C_1$—$C_5$-Alkoxy, Benzyl und den Rest Y substituiertes Phenyl, wobei Y ein gegebenenfalls mit $C_1$—$C_3$-Alkyl, $C_1$—$C_3$-Alkoxy, $C_1$—$C_3$-Halogenalkyl oder Halogen substituiertes Benzoyl darstellt; oder

c) $R_3$, $R_4$, $R_5$ und X die für Formel I angegebenen Bedeutungen haben und einer der Substituenten $R_1$ und $R_2$ für $C_1$—$C_3$-Alkoxy und der andere für $C_1$—$C_5$-Alkyl, $C_1$—$C_3$-Alkoxy, $C_3$—$C_6$-Cycloalkyl, Allyl oder Phenyl steht; oder

d) $R_1$, $R_2$, $R_3$ und X die für Formel I angegebenen Bedeutungen haben und einer der Substituenten $R_4$ und $R_5$ für $C_2$—$C_5$-Alkoxy oder $C_1$—$C_5$-Alkylthio und der andere für Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio steht; oder

e) $R_3$, $R_4$, $R_5$ und X die für Formel I angegebenen Bedeutungen haben und einer der Substituenten $R_1$ und $R_2$ für Alkyl mit 5 Kohlenstoffatomen und der andere für $C_4$—$C_6$-Cycloalkyl oder Phenyl steht; oder

f) $R_1$, $R_2$, $R_3$ und X die für Formel I angegebenen Bedeutungen haben und einer der Substituenten $R_4$ und $R_5$ für in m- oder p-Stellung zur Amidgruppe befindliches Alkyl mit 5 Kohlenstoffatomen steht und der andere Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio bedeutet; oder

g) $R_1$, $R_2$, $R_3$ und X die für Formel I angegebenen Bedeutungen haben und einer der Substituenten $R_4$ und $R_5$ für in o-Stellung zur Amidgruppe befindliches Alkyl mit 5 Kohlenstoffatomen und der andere Halogen, $C_1$—$C_5$-Halogenalkyl, $C_1$—$C_5$-Alkoxy oder $C_1$—$C_5$-Alkylthio bedeutet; oder

h) $R_1$, $R_2$, $R_3$ und X die für Formel I angegebenen Bedeutungen haben, einer der Substituenten $R_4$ und $R_5$ für in o- Stellung zur Amidgruppe befindliches Alkyl mit 5 Kohlenstoffatomen steht und der andere Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio bedeutet; und das Strukturelement —X—$R_3$, wenn es 4-Trifluormethylphenoxy repräsentiert, in o- oder m-Stellung zur Amidgruppe steht; oder

i) einer der Substituenten $R_1$ und $R_2$ für $C_4$—$C_6$-Cycloalkyl oder Phenyl und der andere für $C_1$—$C_5$-Alkyl, $C_1$—$C_5$-Alkoxy, $C_3$—$C_6$-Cycloalkyl, Allyl oder Phenyl steht, einer der Substituenten $R_4$ und $R_5$ für in o-Stellung zur Amidgruppe befindliches Alkyl mit 5 Kohlenstoffatomen und der andere für Wasserstoff, Halogen, $C_1$—$C_5$-Halogenalkyl, $C_1$—$C_5$-Alkyl, $C_1$—$C_5$-Alkoxy oder $C_1$—$C_5$-Alkylthio steht und das Strukturelement —X—$R_3$ 4-Trifluormethylphenoxy repräsentiert, dadurch gekennzeichnet, dass man

a) bei Temperaturen von 50° bis 250°C einen Ester der Formel

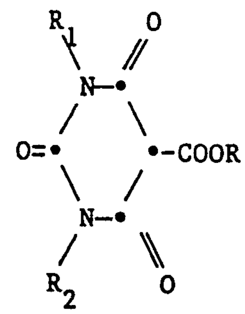

(II)

mit einem Anilinderivat der Formel III

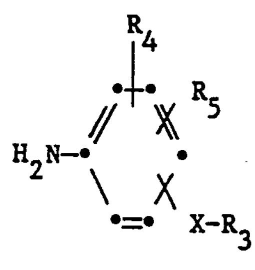

(III)

worin R eine Niederalkyl- oder eine gegebenenfalls durch Nitro substituierte Phenylgruppe darstellt und die Reste $R_1$ bis $R_5$ und X die unter der Formel I in Anspruch 1 angegebenen Bedeutungen besitzen, umsetzt oder

b) bei Temperaturen von 0° bis 220°C eine substituierte Barbitursäure der Formel IV

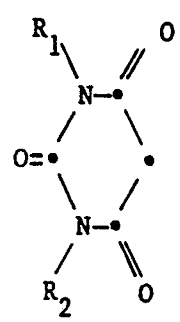

(IV)

mit einem substituierten Phenylisocyanat der Formel V

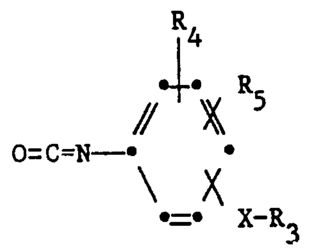

(V)

worin die Reste $R_1$ bis $R_5$ und X die unter Formel I in Anspruch 1 angegebenen Bedeutungen besitzen, umsetzt oder

c) bei Temperaturen von 50° bis 250°C eine substituierte Barbitursäure der Formel IV

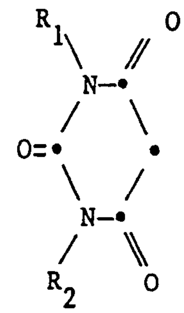

(IV)

mit einem substituierten Benzoylazid der Formel

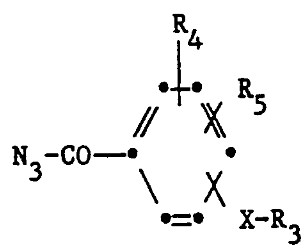

(VI)

worin die Reste $R_1$ bis $R_5$ und X die unter Formel I in Anspruch 1 angegebenen Bedeutungen besitzen, umsetzt.

2. Verfahren nach Anspruch 1, dadurch gekennzeichnet, dass die Verfahren (a), (b) und (c) in Gegenwart reaktionsinerter Lösungs- oder Verdünnungsmittel durchgeführt werden.

3. Verfahren nach den Ansprüchen 1 und 2, dadurch gekennzeichnet, dass die Verfahren (a) und (c) bei 100° bis 220°C und Verfahren (b) bei 0° bis 200°C durchgeführt werden.

4. Verfahren nach den Ansprüchen 2 und 3, dadurch gekennzeichnet, dass die Verfahren (a), (b) und (c) in Gegenwart einer Base durch geführt werden.

5. Verfahren gemäss Anspruch 1 zur Herstellung von 1,3-Dimethyl-5-[4-(3,5-dichlor-pyridyl-2-oxy)-phenylcarbamoyl]-barbitursäure.

6. Verfahren gemäss Anspruch 1 zur Herstellung von 1,3-Dimethyl-5-[4-(5-trifluormethyl-pyridyl-2-oxy)-phenylcarbamoyl]-barbitursäure.

7. Verfahren gemäss Anspruch 1 zur Herstellung von 1,3-Dimethyl-5-[3,5-dichlor-4-(3-chlor-5-trifluormethyl-pyridyl-2-oxy)-phenylcarbamoyl]-barbitursäure.

8. Verfahren gemäss Anspruch 1 zur Herstellung von 1,3-Dimethyl-5-[3,5-dichlor-4-(5-trifluormethyl-pyridyl-2-oxy)-phenylcarbamoyl]-barbitursäure.

9. Verfahren gemäss Anspruch 1 zur Herstellung von 1-Methyl-3-allyl-5-[4-(5-trifluormethyl-pyridyl-2-oxy)-phenylcarbamoyl]-barbitursäure.

10. Verfahren gemäss Anspruch 1 zur Herstellung von 1-Methyl-3-cyclopropyl-5-[4-(5-trifluormethyl-pyridyl-2-oxy)-phenylcarbamoyl]-barbitursäure.

11. Anthelmintisches Mittel, dadurch gekennzeichnet, dass es als aktive Komponente mindestens eine Verbindung der Formel I, ein Tautomeres oder ein Salz davon gemäss Anspruch 1 neben Träger- und weiteren Hilfsstoffen enthält.

12. Mittel nach Anspruch 11, dadurch gekennzeichnet, dass es einen Wirkstoffgehalt von 0,1 bis 99,0 Gew.% und einen Gehalt an Träger- und weiteren Hilfsstoffen von 99,9 bis 1 Gew.% besitzt.

13. Verbindung der Formel I gemäss Anspruch 1 zur Anwendung bei der Bekämpfung parasitärer Helminthen.

14. Verwendung einer Verbindung der Formel I gemäss Anspruch 1 zur Herstellung eines Arzneimittels zur Bekämpfung tierparasitärer Helminthen.

**Revendications pour les Etats contractants: BE CH DE FR IT LI NL SE**

1. Dérivés de l'acide 5-phénylcarbamoylbarbiturique de formule générale I ci-dessous:

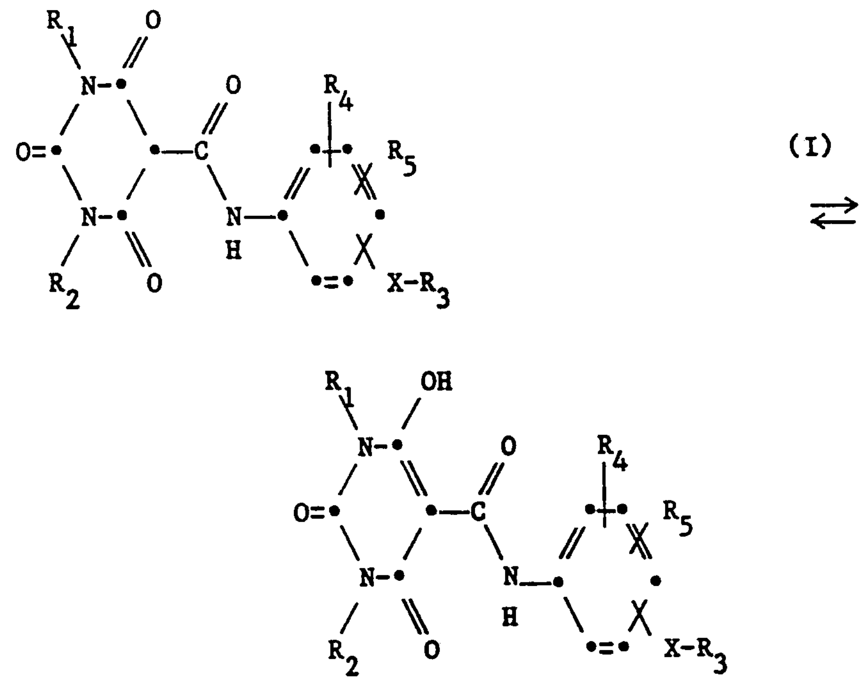

(I)

ainsi que les formes tautomères et sels de ces composés, formule dans laquelle:

$R_1$ et $R_2$ représentent chacun, indépendamment l'un de l'autre, un alkyle en $C_1$—$C_5$, un alcoxy en $C_1$—$C_3$, un cycloalkyle en $C_3$—$C_6$, un allyle ou un phényle;

$R_3$ désigne le groupe phényle, naphtyle ou pyridyle pouvant avoir chacun éventuellement de 1 à 3 substituants pris parmi les halogènes et les groupes cyano, thiocyano, isothiocyano, $C_1$—$C_5$-alcanoyles, $C_1$—$C_5$-alkyles, $C_1$—$C_5$-halogénoalkyles, $C_1$—$C_5$-halogénoalcoxy, $C_1$—$C_5$-alcoxy et benzyle et/ou le radical Y qui est un groupe benzoyle éventuellement substitué par un $C_1$—$C_3$-alkyle, $C_1$—$C_3$-alcoxy ou $C_1$—$C_3$-halogénoalkyle ou par un halogène;

$R_4$ et $R_5$ représentent chacun, indépendamment l'un de l'autre, l'hydrogène ou un halogène, ou un groupe $C_1$—$C_5$-halogénoalkyle, $C_1$—$C_5$-alkyle, $C_1$—$C_5$-alcoxy ou $C_1$—$C_5$-alkylthio; et

X symbolise l'oxygène ou le soufre, avec la condition que:

a) $R_1$, $R_2$, $R_4$, $R_5$ et X ont les significations indiquées et $R_3$ soit un naphtyle ou un pyridyle pouvant avoir chacun éventuellement de 1 à 3 substituants pris parmi les halogènes et les groupes cyano, thiocyano, isothiocyano, $C_1$—$C_5$-alcanoyles, $C_1$—$C_5$-alkyles, $C_1$—$C_5$-halogénoalkyles, $C_1$—$C_5$-halogénoalcoxy, $C_1$—$C_5$-alcoxy et benzyle et/ou le radical Y qui est un radical benzoyle éventuellement substitué par un $C_1$—$C_3$-alkyle, un $C_1$—$C_3$-alcoxy, un $C_1$—$C_3$-halogénoalkyle ou un halogène; ou bien que

b) $R_1$, $R_2$, $R_4$, $R_5$ et X ont les significations indiquées et $R_3$ soit un phényle avec un substituant pris parmi les groupes cyano, thiocyano, isothiocyano, $C_1$—$C_5$-alcanoyles, $C_1$—$C_5$-halogénoalcoxy, $C_1$—$C_5$-alcoxy et benzyle ou avec en plus un ou deux substituants pris parmi les halogènes, les groupes cyano, thiocyano et isothiocyano, $C_1$—$C_5$-alcanoyles, $C_1$—$C_5$-alkyles, $C_1$—$C_5$-halogénoalkyles, $C_1$—$C_5$-halogénoalcoxy, $C_1$—$C_5$-alcoxy et benzyle et le radical Y qui est un radical benzoyle éventuellement substitué par un $C_1$—$C_3$-alkyle, un $C_1$—$C_3$-alcoxy, un $C_1$—$C_3$-halogénoalkyle ou un halogène; ou bien que:

c) $R_3$, $R_4$, $R_5$ et X ont les significations indiquées et l'un des substituants $R_1$ et $R_2$ est un alcoxy en $C_1$—$C_3$ et l'autre un alkyle en $C_1$—$C_5$, un alcoxy en $C_1$—$C_3$, un cycloalkyle en $C_3$—$C_6$, un allyle ou un phényle; ou bien que

d) $R_1$, $R_2$, $R_3$ et X ont les signfications indiquées et l'un des substituants $R_4$ et $R_5$ est un alcoxy en $C_1$—$C_5$ ou un alkylthio en $C_1$—$C_5$ et l'autre l'hydrogène ou un halogène ou un $C_1$—$C_5$-halogénoalkyle, un $C_1$—$C_5$-alkyle, un $C_1$—$C_5$-alcoxy ou un $C_1$—$C_5$-alkylthio; ou bien que:

e) $R_3$, $R_4$, $R_5$ et X ont les significations indiquées et l'un des substituants $R_1$ et $R_2$ est un alkyle à 5 atomes de carbone et l'autre un cycloalkyle en $C_4$—$C_6$ ou un phényle, ou bien que:

f) $R_1$, $R_2$, $R_3$ et X ont les significations indiquées et l'un des substituants $R_4$ et $R_5$ est un alkyle à 5 atomes de carbone à la position m ou p par rapport au groupe amido et l'autre l'hydrogène ou un halogène ou un $C_1$—$C_5$-halogénoalkyle, un $C_1$—$C_5$-alkyle, un $C_1$—$C_5$-alcoxy ou un $C_1$—$C_5$-alkylthio; ou bien que:

g) $R_1$, $R_2$, $R_3$ et X ont les significations indiquées et l'un des substituants $R_4$ et $R_5$ est un alkyle à 5 atomes de carbone à la position o par rapport au groupe amido et l'autre un halogène ou un $C_1$—$C_5$-halogénoalkyle, un $C_1$—$C_5$-alcoxy ou un $C_1$—$C_5$-alkylthio; ou bien que:

h) $R_1$, $R_2$, $R_3$ et X ont les signfications indiquées et l'un des substituants $R_4$ et $R_5$ est un alkyle à 5 atomes de carbone à la position o par rapport au groupe amido et l'autre un hydrogène, un halogène ou un $C_1$—$C_5$-halogénoalkyle, un $C_1$—$C_5$-alkyle, un $C_1$—$C_5$-alcoxy ou un $C_1$—$C_5$-alkylthio, et que le groupe —X—$R_3$, s'il s'agit du groupe 4-trifluorométhylephénoxy, soit à la position ortho ou méta par rapport au groupe amido; ou encore que:

i) l'un des substituants $R_1$ ou $R_2$ soit un cycloalkyle en $C_4$—$C_6$ ou un phényle et l'autre un $C_1$—$C_5$-alkyle, un $C_1$—$C_5$-alcoxy, un $C_3$—$C_6$-cycloalkyle, un allyle ou un phényle, que l'un des substituants $R_4$ et $R_5$ soit un alkyle à 5 atomes de carbone en position ortho par rapport au groupe amido et l'autre l'hydrogène, un halogène ou un $C_1$—$C_5$-halogénoalkyle, un $C_1$—$C_5$-alkyle, un $C_1$—$C_5$-alcoxy ou un $C_1$—$C_5$-alkylthio, et encore que le groupe —X—$R_3$ soit le groupe 4-trifluorométhylphénoxy.

2. Acide 1,3-diméthyl-5-[4-(3,5-dichloro-pyridyl-2-oxy)-phényl-carbamoyl]-barbiturique.

3. Acide 1,3-diméthyl-5-[4-(5-trifluoro-pyridyl-2-oxy)-phényl-carbamoyl]-barbiturique.

4. Acide 1,3-diméthyl-5-[3,5-dichloro-4-(3-chloro-5-trifluorométhyl-pyridyl-2-oxy)-phénylcarbamoyl]-barbiturique.

5. Acide 1,3-diméthyl-5-[3,5-dichloro-4-(5-trifluorométhyl-pyridyl-2-oxy)-phénylcarbamoyl]-barbiturique.

6. Acide 1-méthyl-3-allyl-5-[4-(5-trifluorométhyl-pyridyl-2-oxy)-phénylcarbamoyl]-barbiturique.

7. Acide 1-méthyl-3-cyclopropyl-5-[4-(5-trifluorométhyl-pyridyl-2-oxy)-phénylcarbamoyl]-barbiturique.

8. Procédé de préparation des composés de formule I selon la revendication 1, procédé caractérisé en ce que:

a) on fait réagir à des températures de 50 à 250°C un ester de formule:

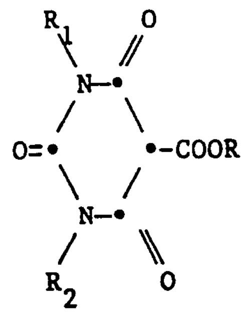

(II)

avec un dérivé d'aniline de formule III:

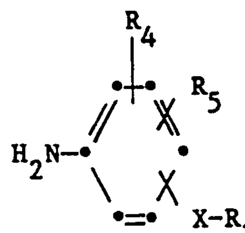

(III)

R étant un alkyle inférieur ou un phényle éventuellement nitré et les autres symboles ayant les signfications données pour la formule I, ou bien

b) on fait réagir à des températures de 0 à 220°C un dérivé de substitution de l'acide barbiturique de formule IV:

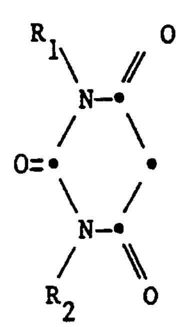

(IV)

avec un isocyanate de phényle de formule V:

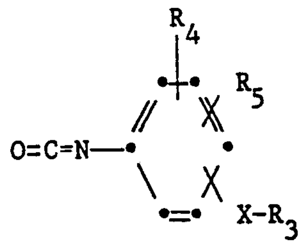

(V)

ou encore

c) on fait réagir à des températures de 50 à 250°C un acide barbiturique de formule IV:

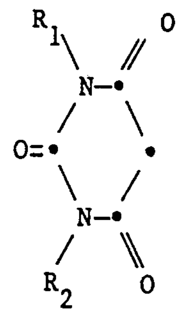

(IV)

avec un benzoylazide de formule:

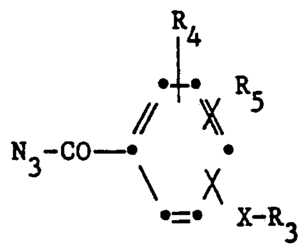

(VI)

9. Procédé selon la revendication 8, caractérisé en ce que l'on exécute les variantes (a), (b) et (c) en présence de solvants ou diluants inertes dans les conditions de la réaction.

10. Procédé selon les revendications 8 et 9, caractérisé en ce que l'on exécute les variantes (a) et (c) entre 100 et 220°C et la variante (b) entre 0 et 200°C.

11. Procédé selon les revendications 9 et 10, caractérisé en ce que l'on exécute les variantes (a), (b) et (c) en présence d'une base.

12. Produit anthelmintique caractérisé en ce qu'il comprend comme composant actif un ou plusieurs composés de formule I selon la revendication 1 ou leurs formes tautomères ou sels, avec des véhicules et autres adjuvants.

13. Produit selon la revendication 12, caractérisé par un teneur en matière active de 0,1 à 99,0% en poids et une teneur en véhicule et autres adjuvants de 99,9 à 1% en poids.

14. Les composés de formule I selon la revendication 1 pour leur emploi contre des helminthes parasitaires.

15. Emploi des composés de formule I selon la revendication 1 pour la fabrication de médicaments destinés à combattre des helminthes qui parasitent des animaux.

**Revendications pour l'Etat contractant: AT**

1. Procédé de préparation d'acides 5-phénylcarbamoyl-barbituriques de formule générale I ci-essous:

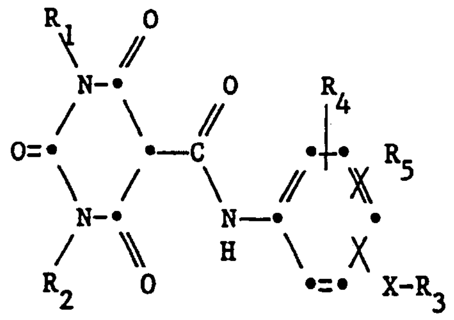

(I)

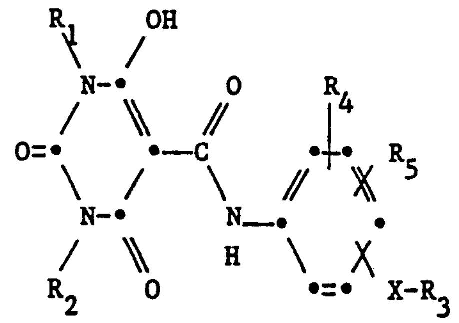

ainsi que des formes tautomères et sels de ces composés, formule dans laquelle:

$R_1$ et $R_2$ représentent chacun, indépendamment l'un de l'autre, un alkyle en $C_1$—$C_5$, un alcoxy en $C_1$—$C_3$, un cycloalkyle en $C_3$—$C_6$, un allyle ou un phényle;

$R_3$ désigne le groupe phényle, naphtyle ou pyridyle pouvant avoir chacun éventuellement de 1 à 3 substituants pris parmi les halogènes et les groupes cyano, thiocyano, isothiocyano, $C_1$—$C_5$-alcanoyles, $C_1$—$C_5$-alkyles, $C_1$—$C_5$-halogénoalkyles, $C_1$—$C_5$-halogénoalcoxy, $C_1$—$C_5$-alcoxy et benzyle et/ou le radical Y qui est un groupe benzoyle éventuellement substitué par un $C_1$—$C_3$-alkyle, $C_1$—$C_3$-alcoxy ou $C_1$—$C_3$-halogénoalkyle ou par un halogène;

$R_4$ et $R_5$ représentent chacun, indépendamment l'un de l'autre, l'hydrogène ou un halogène, ou un groupe $C_1$—$C_5$-halogénoalkyle, $C_1$—$C_5$-alkyle, $C_1$—$C_5$-alcoxy ou $C_1$—$C_5$-alkylthio; et

X symbolise l'oxygène ou le soufre, acec la condition que:

a) $R_1$, $R_2$, $R_4$, $R_5$ et X ont les significations indiquées et $R_3$ soit un naphtyle ou un pyridyle pouvant avoir chacun éventuellement de 1 à 3 substituants pris parmi les halogènes et les groupes cyano, thiocyano, isothiocyano, $C_1$—$C_5$-alcanoyles, $C_1$—$C_5$-alkyles, $C_1$—$C_5$-halogénoalkyles, $C_1$—$C_5$-halogénoalcoxy, $C_1$—$C_5$-alcoxy et benzyle et/ou le radical Y qui est un radical benzoyle éventuellement substitué par un $C_1$—$C_3$-alkyle, un $C_1$—$C_3$-alcoxy, un $C_1$—$C_3$-halogénoalkyle ou un halogène; ou bien que

b) $R_1$, $R_2$, $R_4$, $R_5$ et X ont les significations indiquées et $R_3$ soit un phényle avec un substituant pris parmi les groupes cyano, thiocyano, isothiocyano, $C_1$—$C_5$-alcanoyles, $C_1$—$C_5$-halogénoalcoxy, $C_1$—$C_5$-alcoxy et benzyle ou avec en plus un ou deux substituants pris parmi les halogènes, les groupes cyano, thiocyano et isothiocyano, $C_1$—$C_5$-alcanoyles, $C_1$—$C_5$-alkyles, $C_1$—$C_5$-halogénoalkyles, $C_1$—$C_5$-halogénoalcoxy, $C_1$—$C_5$-alcoxy et benzyle et le radical Y qui est un radical benzoyle éventuellement substitué par un $C_1$—$C_3$-alkyle, un $C_1$—$C_3$-alcoxy, un $C_1$—$C_3$-halogénoalkyle ou un halogène; ou bien que:

c) $R_3$, $R_4$, $R_5$ et X ont les significations indiquées et l'un des substituants $R_1$ et $R_2$ est un alcoxy en $C_1$—$C_3$ et l'autre un alkyle en $C_1$—$C_5$, un alcoxy en $C_1$—$C_3$, un cycloalkyle en $C_3$—$C_6$, un allyle ou un phényle; ou bien que

d) $R_1$, $R_2$, $R_3$ et X ont les signfications indiquées et l'un des substituants $R_4$ et $R_5$ est un alcoxy en $C_1$—$C_5$ ou un alkylthio en $C_1$—$C_5$ et l'autre l'hydrogène ou un halogène ou un $C_1$—$C_5$-halogénoalkyle, un $C_1$—$C_5$-alkyle, un $C_1$—$C_5$-alcoxy ou un $C_1$—$C_5$-alkylthio; ou bien que:

e) $R_3$, $R_4$, $R_5$ et X ont les significations indiquées et l'un des substituants $R_1$ et $R_2$ est un alkyle à 5 atomes de carbone et l'autre un cycloalkyle en $C_4$—$C_6$ ou un phényle, ou bien que:

f) $R_1$, $R_2$, $R_3$ et X ont les significations indiquées et l'un des substituants $R_4$ et $R_5$ est un alkyle à 5 atomes de carbone à la position m ou p par rapport au groupe amido et l'autre l'hydrogène ou un halogène ou un $C_1$—$C_5$-halogénoalkyle, un $C_1$—$C_5$-alkyle, un $C_1$—$C_5$-alcoxy ou un $C_1$—$C_5$-alkylthio; ou bien que:

g) $R_1$, $R_2$, $R_3$ et X ont les significations indiquées et l'un des substituants $R_4$ et $R_5$ est un alkyle à 5 atomes de carbone à la position o par rapport au groupe amido et l'autre un halogène ou un $C_1$—$C_5$-halogénoalkyle, un $C_1$—$C_5$-alcoxy ou un $C_1$—$C_5$-alkylthio; ou bien que:

h) $R_1$, $R_2$, $R_3$ et X ont les signfications indiquées et l'un des substituants $R_4$ et $R_5$ est un alkyle à 5 atomes de carbone à la position o par rapport au groupe amido et l'autre un hydrogène, un halogène ou un $C_1$—$C_5$-halogénoalkyle, un $C_1$—$C_5$-alkyle, un $C_1$—$C_5$-alcoxy ou un $C_1$—$C_5$-alkylthio, et que le groupe —X—$R_3$, s'il s'agit du groupe 4-trifluorométhylephénoxy, soit à la position ortho ou méta par rapport au groupe amido; ou encore que:

i) l'un des substituants $R_1$ ou $R_2$ soit un cycloalkyle en $C_4$—$C_6$ ou un phényle et l'autre un $C_1$—$C_5$-alkyle, un $C_1$—$C_5$-alcoxy, un $C_3$—$C_6$-cycloalkyle, un allyle ou un phényle, que l'un des substituants $R_4$ et $R_5$ soit un alkyle à 5 atomes de carbone en position ortho par rapport au groupe amido et l'autre l'hydrogène, un halogène ou un $C_1$—$C_5$-halogénoalkyle, un $C_1$—$C_5$-alkyle, un $C_1$—$C_5$-alcoxy ou un $C_1$—$C_5$-alkylthio, et encore que le groupe —X—$R_3$ soit le groupe 4-trifluorométhylphénoxy, procédé caractérisé en ce que:

a) on fait réagir à des températures de 50 à 250°C un ester de formule:

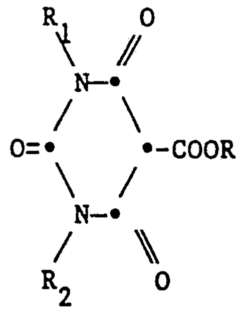

(II)

avec un dérivé d'aniline de formule III:

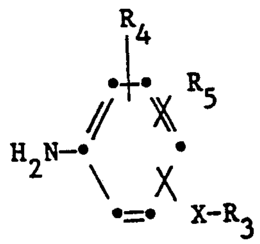

(III)

R étant un alkyle inférieur ou un phényle éventuellement nitré et les autres symboles ayant les signfications données pour la formule I, ou bien

b) on fait réagir à des températures de 0 à 220°C un dérivé de substitution de l'acide barbiturique de formule IV:

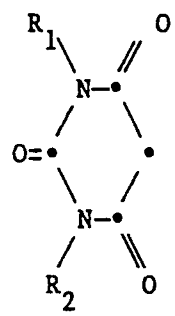

(IV)

avec un isocyanate de phényle de formule V:

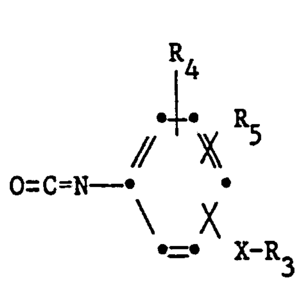

(V)

ou encore

c) on fait réagir à des températures de 50 à 250°C un acide barbiturique de formule IV:

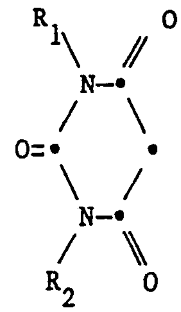

(IV)

avec un benzoylazide de formule:

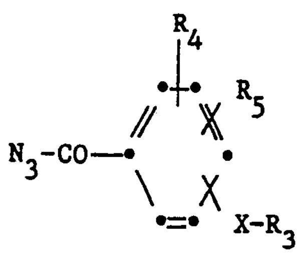

(VI)

2. Procédé selon la revendication 1, caractérisé en ce que l'on exécute les variantes (a), (b) et (c) en présence de solvants ou diluants inertes dans les conditions de la réaction.

3. Procédé selon les revendications 1 et 2, caractérisé en ce que l'on exécute les variantes (a) et (c) entre 100 et 220°C et la variante (b) entre 0 et 200°C.

4. Procédé selon les revendications 2 et 3, caractérisé en ce que l'on exécute les variantes (a), (b) et (c) en présence d'une base.

5. Procédé selon la revendication 1 pour préparer l'acide 1,3-diméthyl-5-[4-(3,5-dichloro-pyridyl-2-oxy)-phénylcarbamoyl]-barbiturique.

6. Procédé selon la revendication 1 pour préparer l'acide 1,3-diméthyl-5-[4-(5-trifluorométhyl-pyridyl-2-oxy)-phénylcarbamoyl]-barbiturique.

7. Procédé selon la revendication 1 pour préparer l'acide 1,3-diméthyl-5-[3,5-dichloro-4-(3-chloro-5-trifluorométhyl-pyridyl-2-oxy)-phénylcarbamoyl]-barbiturique.

8. Procédé selon la revendication 1 pour préparer l'acide 1,3-diméthyl-5-[3,5-dichloro-4-(5-trifluorométhyl-pyridyl-2-oxy)-phénylcarbamoyl]-barbiturique.

9. Procédé selon la revendication 1 pour préparer l'acide 1-méthyl-3-allyl-5-[4-(5-trifluorométhylpyridyl-2-oxy)-phénylcarbamoyl]-barbiturique.

10. Procédé selon la revendication 1 pour préparer l'acide 1-méthyl-3-cyclopropyl-5-[4-(5-trifluorométhyl-pyridyl-2-oxy)-phénylcarbamoyl]-barbiturique.

11. Produit anthelmintique caractérisé en ce qu'il comprend comme composant actif un ou plusieurs composés de formule I selon la revendication 1 ou leurs formes tautomères ou sels, avec des véhicules et autres adjuvants.

12. Produit selon la revendication 11 caractérisé en une teneur en matière active de 0,1 à 99,0% en poids et une teneur en véhicules et autres adjuvants de 99,9 à 1% en poids.

13. Les composés de formule I selon la revendication 1 pour leur emploi dans la lutte contre des helminthes parasitaires.

14. Emploi des composés de formule I selon la revendication 1 pour la fabrication de médicaments destinés à lutter contre des helminthes qui parasitent des animaux.

**Claims for the Contracting States: BE CH DE FR IT LI NL SE**

1. A 5-phenylcarbamoylbarbituric acid derivative of the general formula I

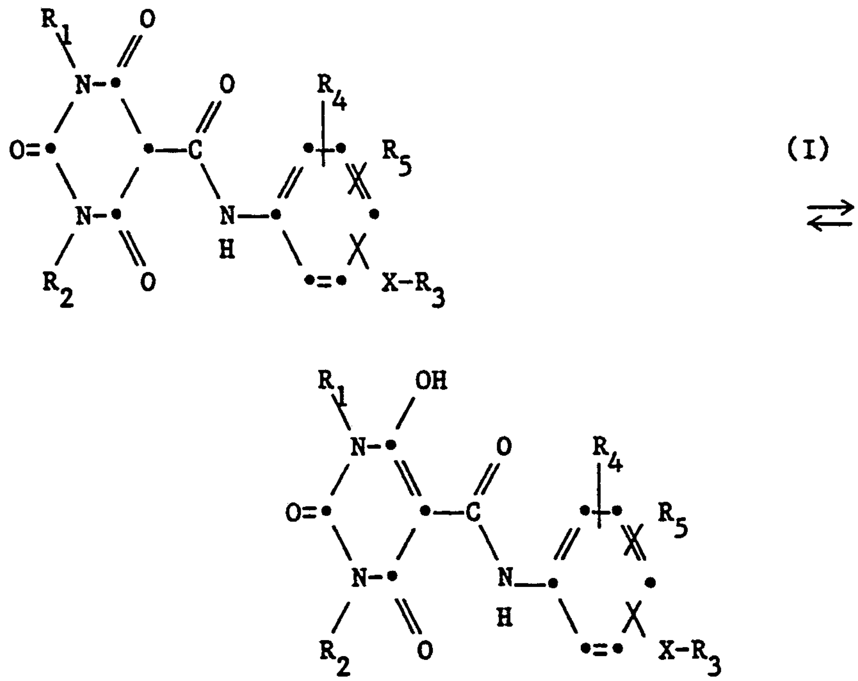

(I)

and tautomeric forms and salts thereof in which $R_1$ and $R_2$ independently of one another are $C_1$—$C_5$ alkyl, $C_1$—$C_3$ alkoxy, $C_3$—$C_6$ cycloalkyl, allyl or phenyl; $R_3$ is phenyl, naphthyl or pyridyl; or $R_3$ is also phenyl or naphthyl or pyridyl each of which is monosubstituted to trisubstituted by halogen, cyano, thiocyano, isothiocyano, $C_1$—$C_5$ alkanoyl, $C_1$—$C_5$ alkyl, $C_1$—$C_5$ halogenoalkyl, $C_1$—$C_5$ halogenoalkoxy, $C_1$—$C_5$ alkoxy, benzyl and/or the radical Y;

Y is benzoyl which is unsubstituted or substituted by $C_1$—$C_3$ alkyl, $C_1$—$C_3$ alkoxy, $C_1$—$C_3$ halogenoalkyl or halogen;

$R_4$ and $R_5$ independently of one another are hydrogen, halogen, $C_1$—$C_5$ halogenoalkyl, $C_1$—$C_5$ alkyl, $C_1$—$C_5$ alkoxy or $C_1$—$C_5$ alkylthio; and

X is oxygen or sulphur, subject to the proviso that

a) $R_1$, $R_2$, $R_4$, $R_5$ and X are as defined for formula I and $R_3$ is naphthyl or pyridyl, or naphthyl or pyridyl each of which is monosubstituted to trisubstituted by halogen, cyano, thiocyano, isothiocyano, $C_1$—$C_5$ alkanoyl, $C_1$—$C_5$ alkyl, $C_1$—$C_5$ halogenoalkyl, $C_1$—$C_5$ halogenoalkoxy, $C_1$—$C_5$ alkoxy, benzyl and/or by the radical Y, Y being a benzoyl which is unsubstituted or substituted by $C_1$—$C_3$ alkyl, $C_1$—$C_3$ alkoxy, $C_1$—$C_3$ halogenoalkyl or halogen; or

b) $R_1$, $R_2$, $R_4$, $R_5$ and X are as defined for formula I and $R_3$ is phenyl which is monosubstituted by cyano, thiocyano, isothiocyano, $C_1$—$C_5$ alkanoyl, $C_1$—$C_5$ halogenoalkoxy, $C_1$—$C_5$ alkoxy, benzyl or by the radical Y or additionally substituted by one or two substituents from the group comprising halogen, cyano, thiocyano, isothiocyano, $C_1$—$C_5$ alkanoyl, $C_1$—$C_5$ alkanoyl, $C_1$—$C_5$ alkyl, $C_1$—$C_5$ halogenoalkyl, $C_1$—$C_5$ halogenalkoxy, $C_1$—$C_5$ alkoxy, benzyl and the radical Y, Y being a benzoyl which is unsubstituted or substituted by $C_1$—$C_3$ alkyl, $C_1$—$C_3$ alkoxy, $C_1$—$C_3$ halogenalkyl or halogen; or

c) $R_3$, $R_4$, $R_5$ and X are as defined for formula I and one of the substituents $R_1$ and $R_2$ is $C_1$—$C_3$ alkoxy and the other is $C_1$—$C_5$ alkyl, $C_1$—$C_3$ alkoxy, $C_3$—$C_6$ cycloalkyl, allyl or phenyl; or

d) $R_1$, $R_2$, $R_3$ and X are as defined for formula I and one of the substituents $R_4$ and $R_5$ is $C_2$—$C_5$ alkoxy or $C_1$—$C_5$ alkylthio and the other is hydrogen, halogen, $C_1$—$C_5$ halogenoalkyl, $C_1$—$C_5$ alkyl, $C_1$—$C_5$ alkoxy or $C_1$—$C_5$ alkylthio; or

e) $R_3$, $R_4$, $R_5$ and X are as defined for formula I and one of the substituents $R_1$ and $R_2$ is alkyl having 5 carbon atoms and the other is $C_4$—$C_6$ cycloalkyl or phenyl; or

f) $R_1$, $R_2$, $R_3$ and X are as defined for formula I and one of the substituents $R_4$ and $R_5$ is alkyl having 5 carbon atoms and situated in the m- or p- position in relation to the amide group and the other is hydrogen, halogen, $C_1$—$C_5$ halogenoalkyl, $C_1$—$C_5$ alkyl, $C_1$—$C_5$ alkoxy or $C_1$—$C_5$ alkylthio; or

g) $R_1$, $R_2$, $R_3$ and X are as defined for formula I and one of the substituents $R_4$ and $R_5$ is alkyl having 5 carbon atoms and situated in the o-position in relation to the amide group and the other is halogen, $C_1$—$C_5$ halogenoalkyl, $C_1$—$C_5$ alkoxy or $C_1$—$C_5$ alkylthio; or

h) $R_1$, $R_2$, $R_3$ and X are as defined for formula I, one of the substituents $R_4$ and $R_5$ is alkyl having 5 carbon atoms and situated in the o-position in relation to the amide group and the other is hydrogen, halogen, $C_1$—$C_5$ halogenoalkyl, $C_1$—$C_5$ alkyl, $C_1$—$C_5$ alkoxy or $C_1$—$C_5$ alkylthio; and the structural element —X—$R_3$ is in the o- or m- position in relation to the amide group, if it represents 4-trifluoromethylphenoxy; or

i) one of the substituents $R_1$ and $R_2$ is $C_4$—$C_6$ cycloalkyl or phenyl and the other is $C_1$—$C_5$ alkyl, $C_1$—$C_5$ alkoxy, $C_3$—$C_6$ cycloalkyl, allyl or phenyl, one of the substituents $R_4$ and $R_5$ is alkyl having 5 carbon atoms and situated in the o-position in relation to the amide group and the other is hydrogen, halogen, $C_1$—$C_5$ halogenoalkyl, $C_1$—$C_5$ alkyl, $C_1$—$C_5$ alkoxy or $C_1$—$C_5$ alkylthio and the structural element —X—$R_3$ represents 4-trifluoromethylphenoxy.

2. 1,3-dimethyl-5-[4-(3,5-dichloropyridyl-2-oxy)-phenylcarbamoyl]-barbituric acid.

3. 1,3-dimethyl-5-[4-(5-trifluoromethylpyridyl-2-oxy)-phenylcarbamoyl]-barbituric acid.

4. 1,3-dimethyl-5-[3,5-dichloro-4-(3-chloro-5-trifluoromethyl-pyridyl-2-oxy)-phenylcarbamoyl]-barbituric acid.

5. 1,3-dimethyl-5-[3,5-dichloro-4-(5-trifluoromethylpyridyl-2-oxy)-phenylcarbamoyl]-barbituric acid.

6. 1-methyl-3-allyl-5-[4-(5-trifluoromethylpyridyl-2-oxy)-phenylcarbamoyl]-barbituric acid.

7. 1-methyl-3-cyclopropyl-5-[4-(5-trifluoromethylpyridyl-2-oxy)-phenylcarbamoyl]-barbituric acid.

8. A process for the preparation of compounds of the formula I according to claim 1, which comprises

a) reacting, at temperatures of 50° to 250°C, an ester of the formula

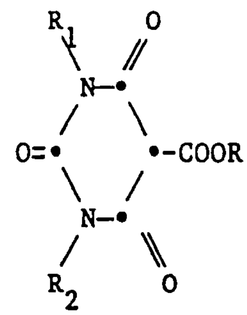

(II)

with an aniline derivative of the formula III

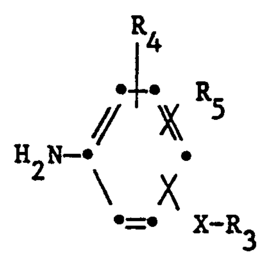

(III)

in which R is a lower alkyl group or a phenyl group which is unsubstituted or substituted by nitro, and the radicals $R_1$ to $R_5$ and X are as defined under formula I in claim 1, or

b) reacting, at temperatures of 0° to 220°C, a substituted barbituric acid of the formula IV

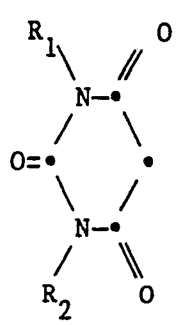

(IV)

with a substituted phenyl isocyanate of the formula V

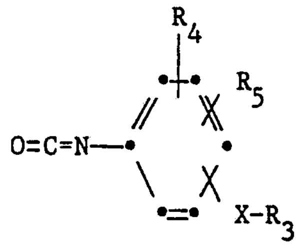

(V)

in which the radicals $R_1$ to $R_5$ and X are as defined under formula I in claim 1, or

c) reacting, at temperatures of 50° to 250°C, a substituted barbituric acid of the formula IV

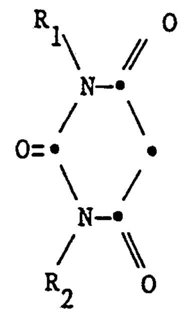

(IV)

with a substituted benzoyl azide of the formula

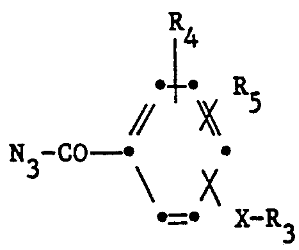

(VI)

in which the radicals $R_1$ to $R_5$ and X are as defined under formula I in claim 1.

9. A process according to claim 8, wherein processes a), b) and c) are carried out in the presence of solvents or diluents which are inert towards the reaction.

10. A process according to either of claims 8 and 9, wherein processes a) and c) are carried out at 100° to 200°C and process b) is carried out at 0° to 200°C.

11. A process according to either of claims 9 and 10, wherein processes a), b) and c) are carried out in the presence of a base.

12. An anthelmintic composition which contains, as the active component, at least one compound of the formula I, a tautomer or a salt therof according to claim 1 and also carriers and further adjuncts.

13. A composition according to claim 12, which contains 0.1 to 99.0% by weight of active compound and 99.9 to 1% by weight of carriers and further adjuncts.

14. A compound of the formula I according to claim 1 for use for controlling parasitic helminths.

15. The use of a compound of the formula I according to claim 1 for the preparation of a medicament for controlling zooparasitic helminths.

**Claims for the Contracting State: AT**

1. A process for the preparation of a 5-phenylcarbamoylbarbituric acid derivative of the general formula I

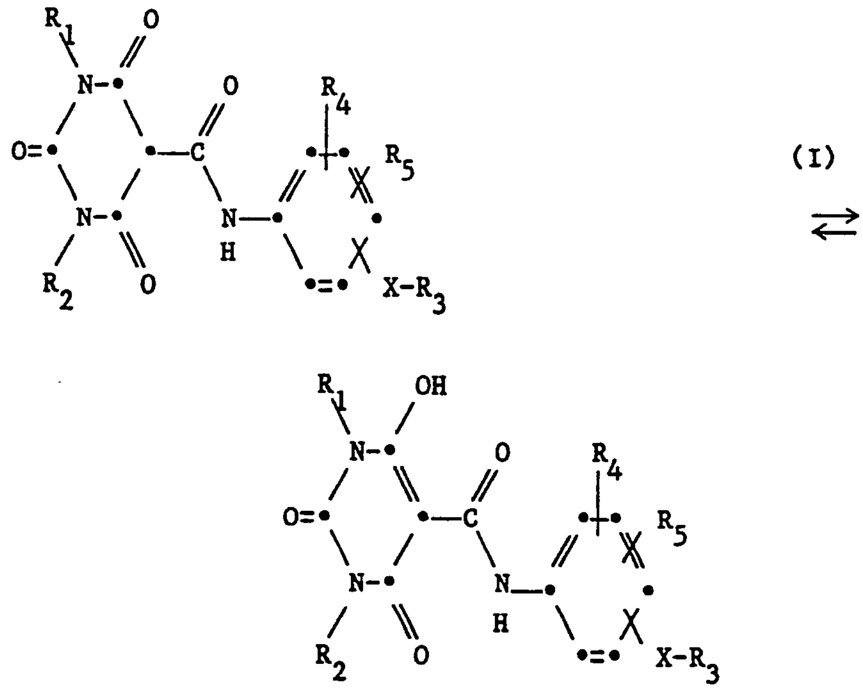

(I)

and tautomeric forms and salts thereof in which $R_1$ and $R_2$ independently of one another are $C_1$—$C_5$ alkyl, $C_1$—$C_3$ alkoxy, $C_3$—$C_6$ cycloalkyl, allyl or phenyl; $R_3$ is phenyl, naphthyl or pyridyl; or $R_3$ is also phenyl or naphthyl or pyridyl each of which is monosubstituted to trisubstituted by halogen, cyano, thiocyano, isothiocyano, $C_1$—$C_5$ alkanoyl, $C_1$—$C_5$ alkyl, $C_1$—$C_5$ halogenoalkyl, $C_1$—$C_5$ halogenoalkoxy, $C_1$—$C_5$ alkoxy, benzyl and/or the radical Y;

Y is benzoyl which is unsubstituted or substituted by $C_1$—$C_3$ alkyl, $C_1$—$C_3$ alkoxy, $C_1$—$C_3$ halogenoalkyl or halogen;

$R_4$ and $R_5$ independently of one another are hydrogen, halogen, $C_1$—$C_5$ halogenoalkyl, $C_1$—$C_5$ alkyl, $C_1$—$C_5$ alkoxy or $C_1$—$C_5$ alkylthio; and

X is oxygen or sulphur, subject to the proviso that

a) $R_1$, $R_2$, $R_4$, $R_5$ and X are as defined for formula I and $R_3$ is naphthyl or pyridyl, or naphthyl or pyridyl each of which is monosubstituted to trisubstituted by halogen, cyano, thiocyano, isothiocyano, $C_1$—$C_5$ alkanoyl, $C_1$—$C_5$ alkyl, $C_1$—$C_5$ halogenoalkyl, $C_1$—$C_5$ halogenoalkoxy, $C_1$—$C_5$ alkoxy, benzyl and/or by the radical Y, Y being a benzoyl which is unsubstituted or substituted by $C_1$—$C_3$ alkyl, $C_1$—$C_3$ alkoxy, $C_1$—$C_3$ halogenoalkyl or halogen; or

b) $R_1$, $R_2$, $R_4$, $R_5$ and X are as defined for formula I and $R_3$ is phenyl which is monosubstituted by cyano, thiocyano, isothiocyano, $C_1$—$C_5$ alkanoyl, $C_1$—$C_5$ halogenoalkoxy, $C_1$—$C_5$ alkoxy, benzyl or by the radical Y or additionally substituted by one or two substituents from the group comprising halogen, cyano, thiocyano, isothiocyano, $C_1$—$C_5$ alkanoyl, $C_1$—$C_5$ alkanoyl, $C_1$—$C_5$ alkyl, $C_1$—$C_5$ halogenoalkyl, $C_1$—$C_5$ halogenalkoxy, $C_1$—$C_5$ alkoxy, benzyl and the radical Y, Y being a benzoyl which is unsubstituted or substituted by $C_1$—$C_3$ alkyl, $C_1$—$C_3$ alkoxy, $C_1$—$C_3$ halogenalkyl or halogen; or

c) $R_3$, $R_4$, $R_5$ and X are as defined for formula I and one of the substituents $R_1$ and $R_2$ is $C_1$—$C_3$ alkoxy and the other is $C_1$—$C_5$ alkyl, $C_1$—$C_3$ alkoxy, $C_3$—$C_6$ cycloalkyl, allyl or phenyl; or

d) $R_1$, $R_2$, $R_3$ and X are as defined for formula I and one of the substituents $R_4$ and $R_5$ is $C_2$—$C_5$ alkoxy or $C_1$—$C_5$ alkylthio and the other is hydrogen, halogen, $C_1$—$C_5$ halogenoalkyl, $C_1$—$C_5$ alkyl, $C_1$—$C_5$ alkoxy or $C_1$—$C_5$ alkylthio; or

e) $R_3$, $R_4$, $R_5$ and X are as defined for formula I and one of the substituents $R_1$ and $R_2$ is alkyl having 5 carbon atoms and the other is $C_4$—$C_6$ cycloalkyl or phenyl; or

f) $R_1$, $R_2$, $R_3$ and X are as defined for formula I and one of the substituents $R_4$ and $R_5$ is alkyl having 5 carbon atoms and situated in the m- or p- position in relation to the amide group and the other is hydrogen, halogen, $C_1$—$C_5$ halogenoalkyl, $C_1$—$C_5$ alkyl, $C_1$—$C_5$ alkoxy or $C_1$—$C_5$ alkylthio; or

g) $R_1$, $R_2$, $R_3$ and X are as defined for formula I and one of the substituents $R_4$ and $R_5$ is alkyl having 5 carbon atoms and situated in the o-position in relation to the amide group and the other is halogen, $C_1$—$C_5$ halogenoalkyl, $C_1$—$C_5$ alkoxy or $C_1$—$C_5$ alkylthio; or

h) $R_1$, $R_2$, $R_3$ and X are as defined for formula I, one of the substituents $R_4$ and $R_5$ is alkyl having 5 carbon atoms and situated in the o-position in relation to the amide group and the other is hydrogen, halogen, $C_1$—$C_5$ halogenoalkyl, $C_1$—$C_5$ alkyl, $C_1$—$C_5$ alkoxy or $C_1$—$C_5$ alkylthio; and the structural element —X—$R_3$ is in the o- or m- position in relation to the amide group, if it represents 4-trifluoromethylphenoxy; or

i) one of the substituents $R_1$ and $R_2$ is $C_4$—$C_6$ cycloalkyl or phenyl and the other is $C_1$—$C_5$ alkyl, $C_1$—$C_5$ alkoxy, $C_3$—$C_6$ cycloalkyl, allyl or phenyl, one of the substituents $R_4$ and $R_5$ is alkyl having 5 carbon atoms and situated in the o- position in relation to the amide group and the other is hydrogen, halogen, $C_1$—$C_5$ halogenoalkyl, $C_1$—$C_5$ alkyl, $C_1$—$C_5$ alkoxy or $C_1$—$C_5$ alkylthio and the structural element —X—$R_3$ represents 4-trifluoromethylphenoxy, which comprises

a) reacting, at temperatures of 50° to 250°C, an ester of the formula

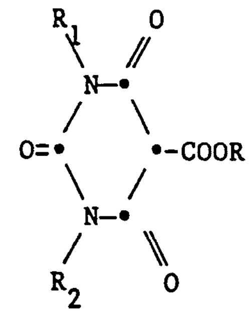

(II)

with an aniline derivative of the formula III

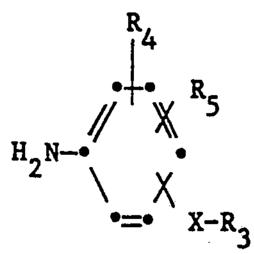

(III)

in which R is a lower alkyl group or a phenyl group which is unsubstituted or substituted by nitro, and the radicals $R_1$ to $R_5$ and X are as defined under formula I in claim 1, or

b) reacting, at temperatures of 0° to 220°C, a substituted barbituric acid of the formula IV

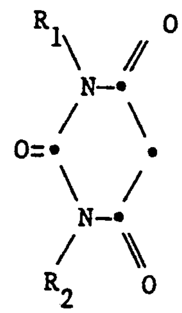

(IV)

with a substituted phenyl isocyanate of the formula V

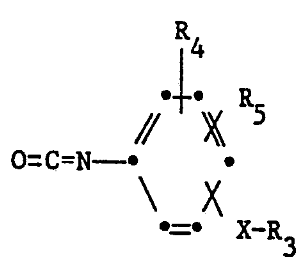

(V)

in which the radicals $R_1$ to $R_5$ and X are as defined under formula I in claim 1, or

c) reacting, at temperatures of 50° to 250°C, a substituted barbituric acid of the formula IV

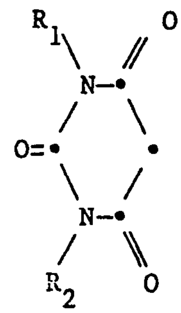

(IV)

with a substituted benzoyl azide of the formula

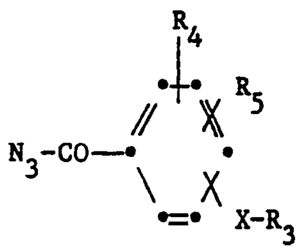

(VI)

in which the radicals $R_1$ to $R_5$ and X are as defined under formula I in claim 1.

2. A process according to claim 1, wherein processes a), b) and c) are carried out in the presence of solvents or diluents which are inert towards the reaction.

3. A process according to either of claims 1 and 2, wherein processes a) and c) are carried out at 100° to 200°C and process b) is carried out at 0° to 200°C.

4. A process according to either of claims 2 and 3, wherein processes a), b) and c) are carried out in the presence of a base.

5. A process according to claim 1 for the preparation of 1,3-dimethyl-5-[4-(3,5-dichloropyridyl-2-oxy)-phenylcarbamoyl]-barbituric acid.

6. A process according to claim 1 for the preparation of 1,3-dimethyl-5-[4-(5-trifluoromethylpyridyl-2-oxy)-phenylcarbamoyl]-barbituric acid.

7. A process according to claim 1 for the preparation of 1,3-dimethyl-5-[3,5-dichloro-4-(3-chloro-5-trifluoromethyl-pyridyl-2-oxy)-phenylcarbamoyl]-barbituric acid.

8. A process according to claim 1 for the preparation of 1,3-dimethyl-5-[3,5-dichloro-4-(5-trifluoromethylpyridyl-2-oxy)-phenylcarbamoyl]-barbituric acid.

9. A process according to claim 1 for the preparation of 1-methyl-3-allyl-5-[4-(5-trifluoromethylpyridyl-2-oxy)-phenylcarbamoyl]-barbituric acid.

10. A process according to claim 1 for the preparation of 1-methyl-3-cyclopropyl-5-[4-(5-trifluoromethylpyridyl-2-oxy)-phenylcarbamoyl]-barbituric acid.

11. An anthelmintic composition which contains, as the active component, at least one compound of the formula I, a tautomer or a salt thereof according to claim 1 and also carriers and further adjuncts.

12. A composition according to claim 11, which contains 0.1 to 99.0% by weight of active compound and 99.9 to 1% by weight of carriers and further adjuncts.

13. A compound of the formula I according to claim 1 for use for controlling parasitic helminths.

14. The use of a compound of the formula I according to claim 1 for the preparation of a medicament for controlling zooparasitic helminths.